# EUROPEAN PATENT APPLICATION

(11) **EP 4 685 137 A1**
(43) Date of publication of application: **28.01.2026**
(21) Application number: 24774095.4
(22) Date of filing: 19.03.2024
(51) Int. Cl.: C07C 317/44, C07C 315/04, A61P 25/00, A61P 39/06, A61P 31/04, A61K 31/192, A61P 13/12, A61P 1/00, A61P 29/00, A61P 11/00, A61P 13/08, A61P 9/00

(54) **SOLID FORM OF NAPHTHYLAMINE MITOPHAGY INDUCER, AND PREPARATION METHOD THEREFOR, PHARMACEUTICAL COMPOSITION THEREOF AND USE THEREOF**

(30) Priority: 21.03.2023 CN 202310280043; 22.03.2023 CN 202310313763
(71) Applicant: Hangzhou Phecdamed Co., Ltd., Hangzhou, Zhejiang 311100 (CN)
(72) Inventor: LIU, Dong, Hangzhou, Zhejiang 311100 (CN); WU, Ronghai, Hangzhou, Zhejiang 311100 (CN); TIAN, Zhen, Hangzhou, Zhejiang 311100 (CN)
(74) Representative: Viering, Jentschura & Partner mbB Patent- und Rechtsanwälte
(86) International application number: PCT/CN2024/082358
(87) International publication number: WO 2024/193530

(57) **Abstract**

The present application discloses solid form of naphthylamine-type mitochondrial autophagy inducer, and preparation method therefor, pharmaceutical composition and use thereof. Various solid forms of the compound of formula (I) are prepared and obtained in the present application, such as sodium salt, potassium salt, calcium salt, tromethamine salt, lysine salt, tert-butylammonium salt, diisopropylammonium salt, ethanolammonium salt, and diethanolammonium salt of the compound of formula (I).

## Description

### Cross-references to Related Applications

This patent application claims priority to Chinese patent application No. 2023102800439, filed on March 21, 2023, entitled " Solid form of naphthylamine-type mitophagy inducer, and preparation method therefor, pharmaceutical composition and use thereof ", which is incorporated herein by reference in its entirety.

This patentapplication claims priority to Chinese patent application No. 2023103137630, filed on March 22, 2023, entitled " Solid form of naphthylamine-type mitophagy inducer, and preparation method therefor, pharmaceutical composition and use thereof ", which is incorporated herein by reference in its entirety.

### Technical field

The present invention relates to the field of chemical drugs, in particular to solid form of naphthylamine-type mitophagy inducer, and preparation method therefor, pharmaceutical composition and use thereof.

### Background technology

Mitochondria are important organelles that regulate a variety of cellular processes and functions, not only as a source of energy in the cell, but also for regulating cell survival and death. Therefore, quality control of mitochondria itself is very important for cells. The quality control of mitochondria is mainly categorized into removal of damaged mitochondria and regulation of nascent mitochondria, and mitophagy, as a selective autophagic process, plays a key role in the removal of damaged mitochondria. The main purpose of mitophagy is to recognize and remove dysfunctional mitochondria. Since mitochondria play a central role in energy supply through oxidation and phosphorylation, having several other important functions including energy metabolism, amino acid production, lipid synthesis, and ionic homeostasis, they are of great importance for maintaining the function of cell types that depend on aerobic metabolism, such as neuronal cells, muscle cells, and liver cells. Homeostatic regulation of mitochondrial production and autophagy is important in maintaining cellular function. Mitophagy dysfunction will lead to accumulation of damaged mitochondria, decreased ability to synthesize ATP+, and generation of large amounts of peroxides, thus causing alteration of cellular intermediary metabolites and triggering a series of pathological consequences. If we enhance mitophagy to remove senescent or dysfunctional mitochondriamitophagy will play a protective role for the cells. Therefore, the development of mitophagy inducers that can effectively induce autophagy in damaged mitochondria, and in particular the one can selectively induce autophagy in damaged mitochondria, is essential for inhibiting or alleviating various acute and chronic diseases caused by mitochondrial dysfunction.

The present applicant has previously developed several compounds having a naphthy lamine structureas mitophagy inducers. Subsequently the applicant further researched the effects of these compounds and selected the efficacious compounds for preparation to obtain the respective solid forms. In the present application, the solid forms of these compounds with the most prospects for pharmaceutical development uses thereof are recorded.

### Summary

It is an object of the present invention to provide a solid form of a compound of formula (I).

It is another object of the present invention to provide a method for preparing the solid form of the compound of formula (I).

It is another object of the present invention to provide a pharmaceutical composition comprising the solid form of the compound of formula (I).

It is another object of the present invention to provide uses thereof the solid form of the compound of formula (I) or the pharmaceutical composition comprising the solid forms of the compounds of formula (I).

In order to solve the above technical problems, the first aspect of the present invention provides a solid form of a pharmaceutically usable salt of a compound of formula (I),

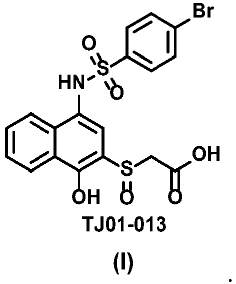

In some preferred embodiments, the pharmaceutically usable salt is a sodium salt, a potassium salt, a calcium salt, a tromethamine salt, a lysine salt, a tert-butylammonium salt, a diisopropylammonium salt, an ethanolammonium salt or a diethanolammonium salt.

In some preferred embodiments, the solid form is a solid form (D crystalline) of a sodium salt of the compound of formula (I), and characteristic peaks of 2θ of 7.06° (±0.2°) and 20.87° (±0.2°) are showed in the X-ray powder diffraction pattern (Cu Kα rays) of the solid form of the sodium salt of the compound of formula (I).

In some preferred embodiments, the solid form of the sodium salt of the compound of formula (I) has a structure as shown in formula (II), 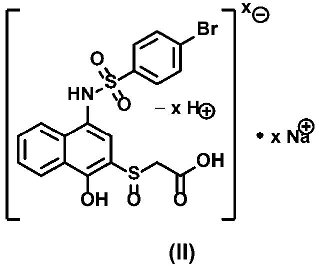 wherein x is selected from 0 .5-2, e.g., 0.5, 0.6, 0.7, 0.8, 0.9, 1.0, 1.1, 1.2, 1.3, 1.4, 1.5, 1.6, 1.7, 1.8, 1.9 or 2.0.

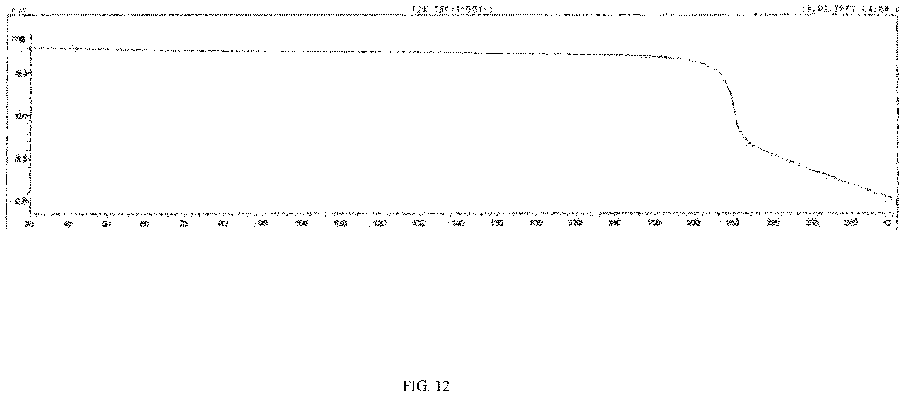

In some preferred embodiments, the solid form of the sodium salt of the compound of formula (I) has at least one of the following characteristics:
the differential scanning calorimetry curve of the solid form of the sodium salt of the compound of formula (I) shows an endothermic peak at 183.79°C (±3°C) and an exothermic peak at 210.79°C (±3°C); and
the solid form of the sodium salt of the compound of formula (I) shows a thermogravimetric analysis curve pattern substantially identical to that of FIG. 12.

In some preferred embodiments, at least one characteristic peak of 2θselected from 7.06° (±0.2°), 18.07° (±0.2°), 25.02° (±0.2°) and 20.87° (±0.2°) is showed in the X-ray powder diffraction pattern of the solid form of the sodium salt of the compound of formula (I).

In some preferred embodiments, at least one characteristicpeakof 2θ selected from 7.06° (±0.2°), 18.07° (±0.2°), 25.02° (±0.2°), 17.58° (±0.2°), 20.87° (±0.2°), 10.54° (±0.2°), 23.91° (±0.2°) is showed the X-ray powder diffraction pattern of the solid form of the sodium salt of the compound of formula (I).

In some preferred embodiments, at least one characteristicpeakof 2θselected from 7.06° (±0.2°), 18.07° (±0.2°), 25.02° (±0.2°), 17.58° (±0.2°), 20.87° (±0.2°), 10.54° (±0.2°), 23.91° (±0.2°), 27.65° (±0.2°), 27.05° (±0.2°), 21.68° (±0.2°), and 25.91° (±0.2°) is showed in the X-ray powder diffraction pattern of the solid form of the sodium salt of the compound of formula (I).

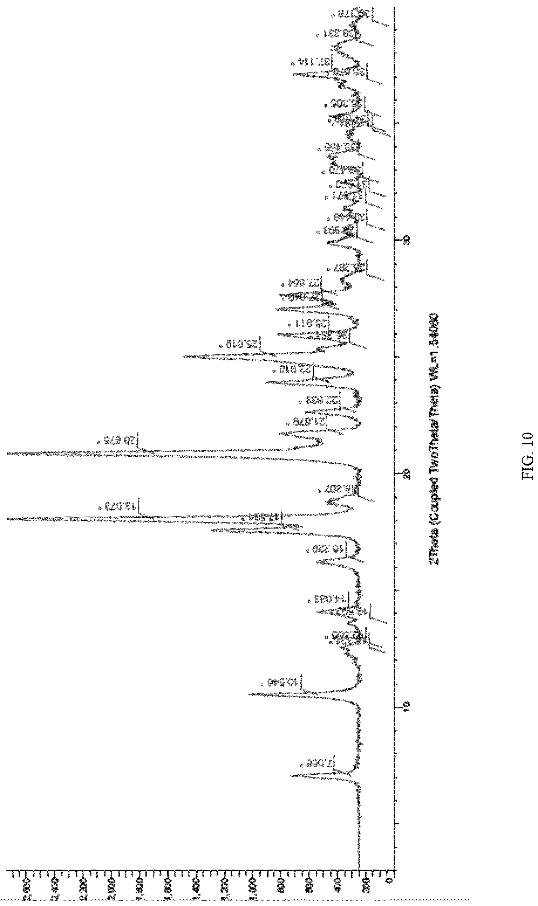

In some preferred embodiments, the solid form of the sodium salt of the compound of formula (I) shows an X-ray powder diffraction pattern substantially identical to that of FIG. 10.

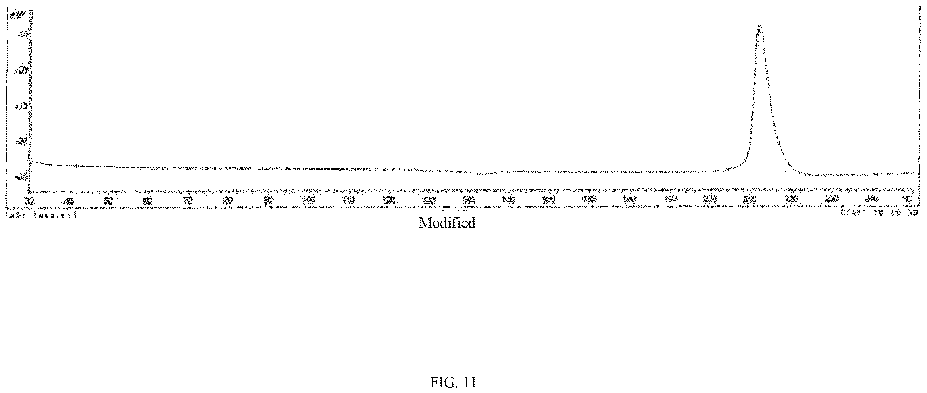

In some preferred embodiments, the solid form of the sodium salt of the compound of formula (I) shows a differential scanning calorimetry curve pattern substantially identical to that of FIG. 11.

In some preferred embodiments, the characteristic peaks of 2θ of 6.85° (±0.2°) and 19.43° (±0.2°) are showed in the X-ray powder diffraction pattern of the solid form (A crystal form) of the sodium salt of the compound of formula (I).

In some preferred embodiments, the solid form (A crystal form) of the sodium salt of the compound of formula (I) has at least one of the following features:
the differential scanning calorimetry curve of the solid form (A crystal form) of the sodium salt of the compound of formula (I) shows an endothermic peak at 144.89°C (±3°C) and 150.40°C (±3°C) and an exothermic peak at 214.79°C (±3°C).

In some preferred embodiments, at least one characteristic peak of 2θ selected from 6.85° (±0.2°), 19.43° (±0.2°), 21.47° (±0.2°) and 4.70° (±0.2°) is showed in the X-ray powder diffraction pattern of the solid form (A crystal form) of the sodium salt of the compound of formula (I).

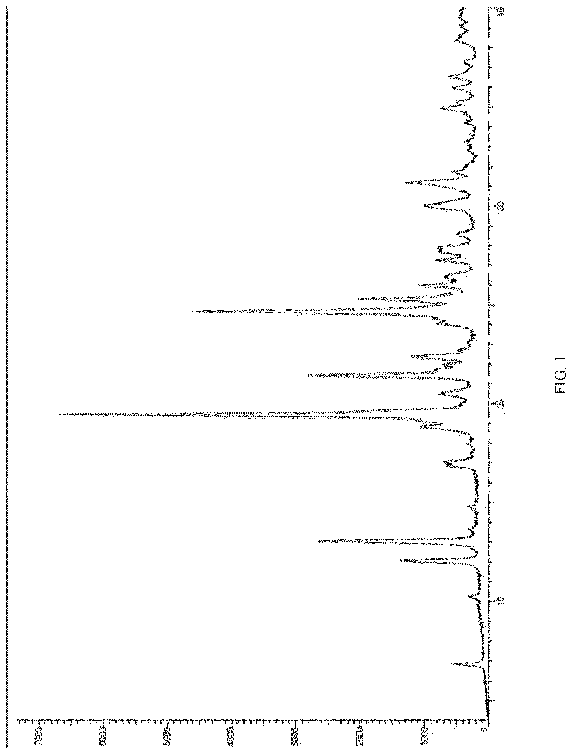

In some preferred embodiments, the solid form of the sodium salt of the compound of formula (I) shows an X-ray powder diffraction pattern substantially identical to that of FIG. 1.

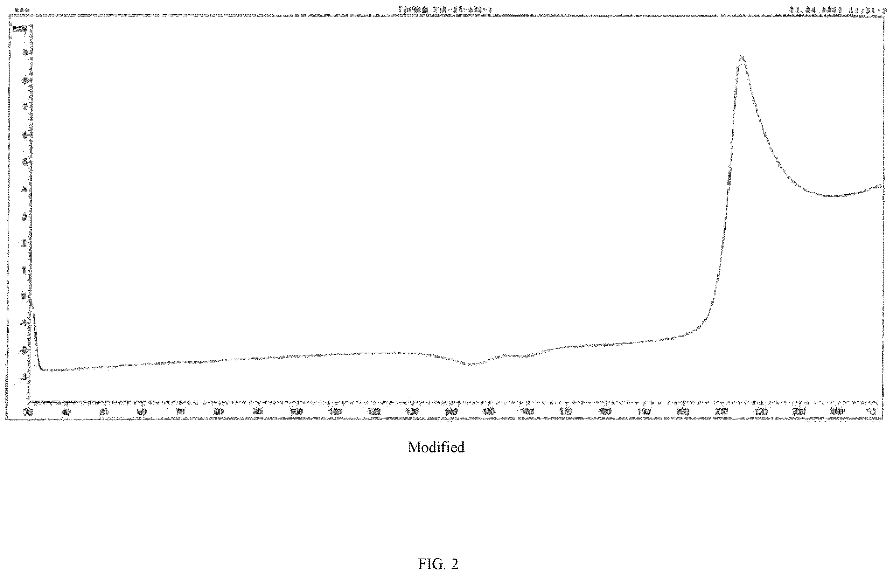

In some preferred embodiments, the solid form of the sodium salt of the compound of formula (I) shows a differential scanning calorimetry curve pattern substantially identical to that of FIG. 2.

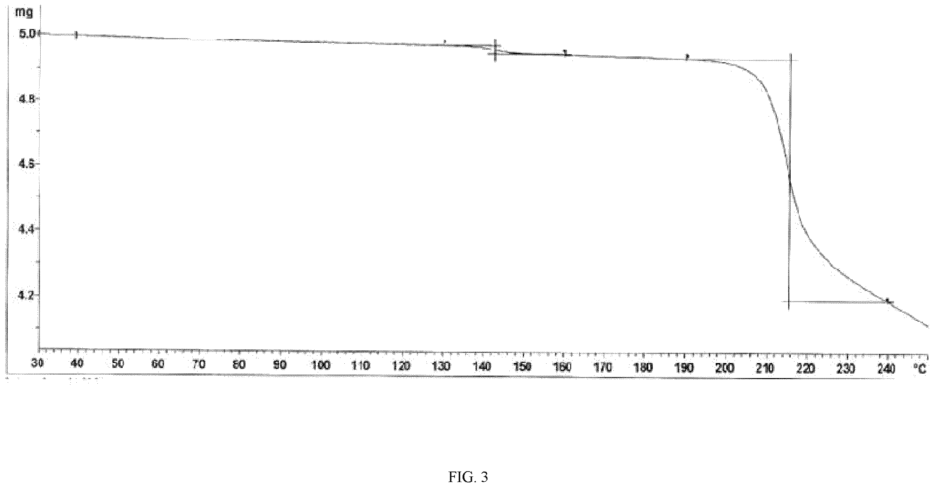

In some preferred embodiments, the solid form of the sodium salt of the compound of formula (I) shows a thermogravimetric analysis curve pattern substantially identical to that of FIG. 3.

In some preferred embodiments, characteristic peaks of 2θ of 7.88° (±0.2°) and 19.29° (±0.2°) are showed in the X-ray powder diffraction pattern of the solid form (B crystal form) of the sodium salt of the compound of formula (I).

In some preferred embodiments, the solid form (B crystal form) of the sodium salt of the compound of formula (I) has at least one of the following features:
the differential scanning calorimetry curve of the solid form of the sodium salt of the compound of formula (I) shows an endothermic peak at 72.14°C (±3°C) and 145.82°C (±3°C) and an exothermic peak at 184.61°C (±3°C); and
the thermogravimetric analysis curve of the solid form of the sodium salt of the compound of formula (I) shows a weight loss peak at 73.50 °C (±3 °C) (preferably, the weight loss is up to 6.78% (±0.2%) relative to the total weight of the solid form).

In some preferred embodiments, at least one characteristic peak of 2θ selected from 7.88° (±0.2°), 9.64° (±0.2°), 14.42° (±0.2°), 19.29° (±0.2°) and 22.65° (±0.2°) is showed in the X-ray powder diffraction pattern of the solid form of the sodium salt of the compound of formula (I).

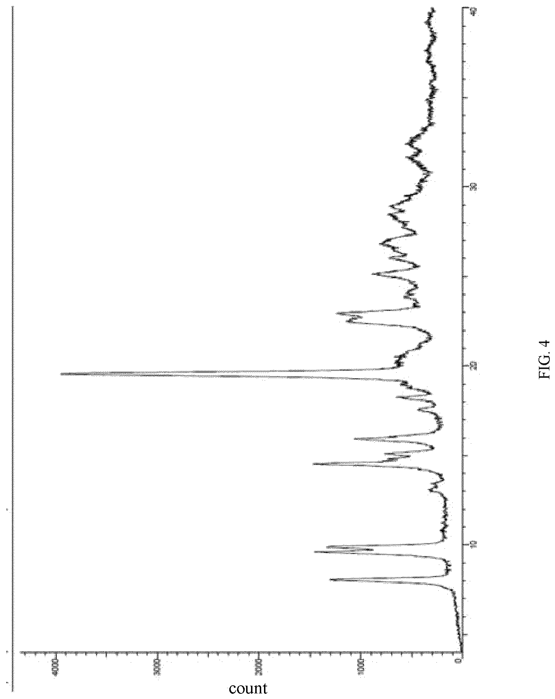

In some preferred embodiments, the solid form of the sodium salt of the compound of formula (I) shows an X-ray powder diffraction pattern substantially identical to that of FIG. 4.

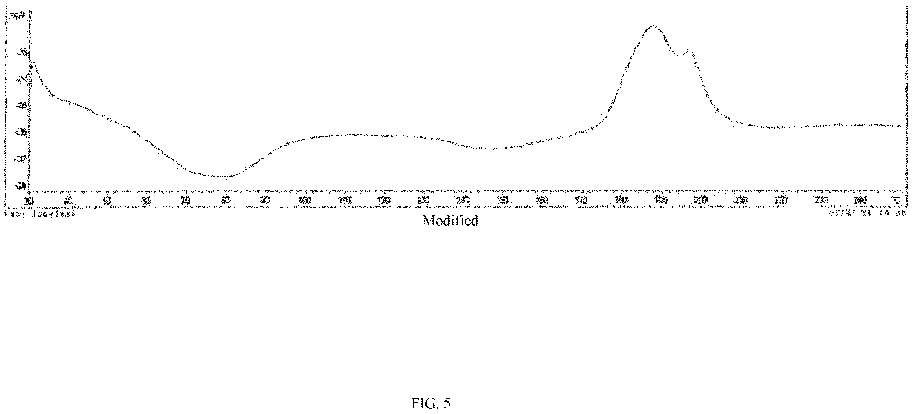

In some preferred embodiments, the solid form of the sodium salt of the compound of formula (I) shows a differential scanning calorimetry curve pattern substantially identical to that of FIG. 5.

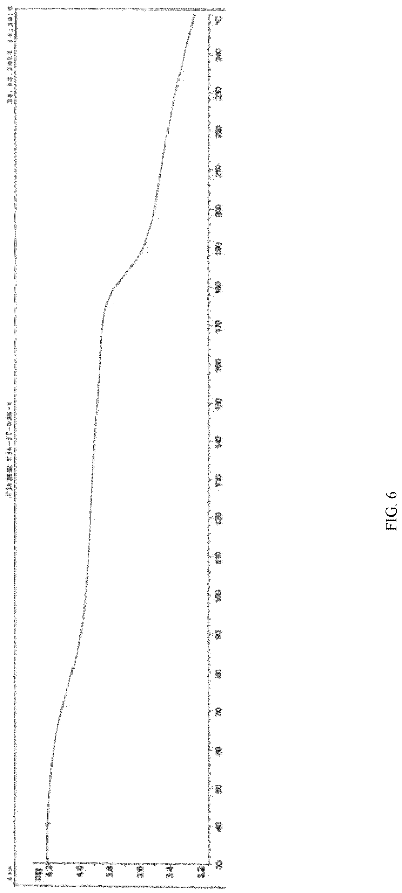

In some preferred embodiments, the solid form of the sodium salt of the compound of formula (I) shows a thermogravimetric analysis curve pattern substantially identical to that of FIG. 6.

In some preferred embodiments, characteristic peaks of 2θ of 8.56° (±0.2°) and 19.96° (±0.2°) are showed in the X-ray powder diffraction pattern of the solid form (C crystal form) of the sodium salt of the compound of formula (I).

In some preferred embodiments, the solid form (C crystal form) of the sodium salt of the compound of formula (I) has at least one of the following features:
the differential scanning calorimetry curve of the solid form of the sodium salt of the compound of formula (I) shows an endothermic peak at 59.75°C (±3°C) and 140.91°C (±3°C) and an exothermic peak at 185.61°C (±3°C); and
the thermogravimetric analysis curve of the solid form of the sodium salt of the compound of formula (I) shows a weight loss peak at 70.15°C (±3°C) (preferably, the weight loss is up to 4.96% (±0.2%) relative to the total weight of the solid form).

In some preferred embodiments, at least one characteristic peak of 2θ selected from 8.56° (±0.2°), 13.24° (±0.2°), 14.62° (±0.2°), 19.96° (±0.2°) and 24.542° (±0.2°) is showed in the X-ray powder diffraction pattern of the solid form of the sodium salt of the compound of formula (I).

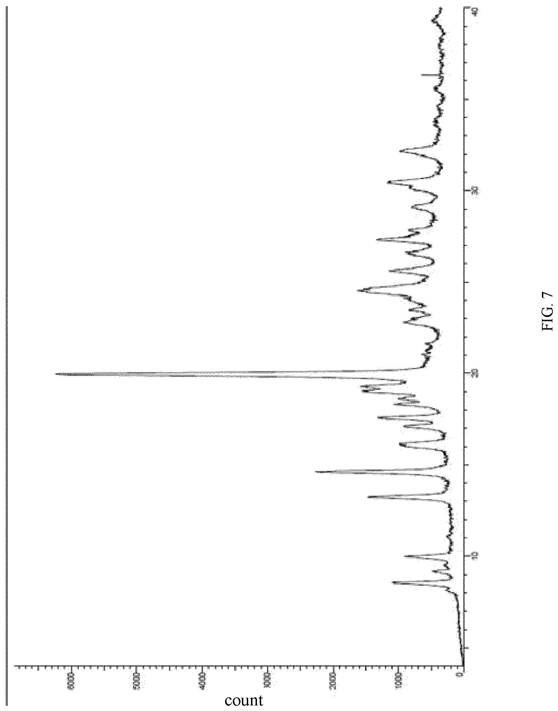

In some preferred embodiments, the solid form of the sodium salt of the compound of formula (I) shows an X-ray powder diffraction pattern substantially identical to that of FIG. 7.

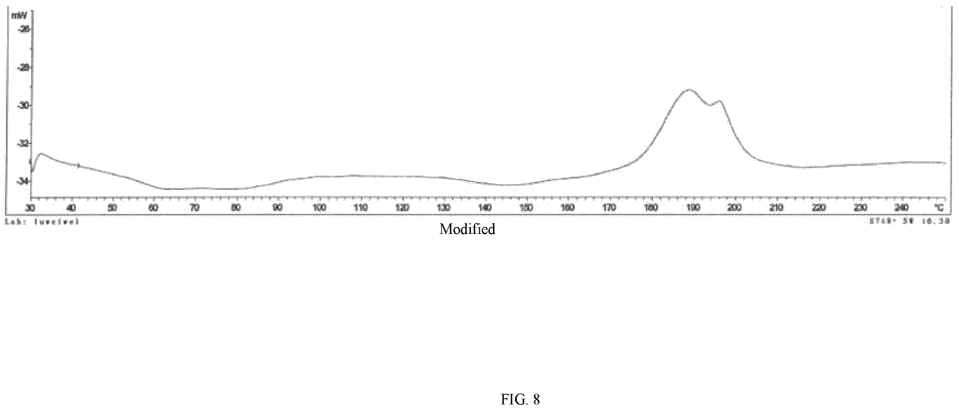

In some preferred embodiments, the solid form of the sodium salt of the compound of formula (I) shows a differential scanning calorimetry curve pattern substantially identical to that of FIG. 8.

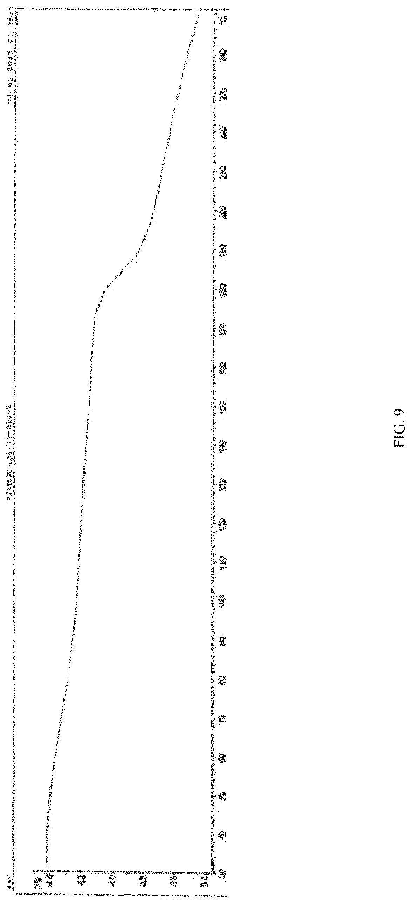

In some preferred embodiments, the solid form of the sodium salt of the compound of formula (I) shows a thermogravimetric analysis curve pattern substantially identical to that of FIG. 9.

In some preferred embodiments, the solid form is a solid form of a potassium salt of the compound of formula (I), and at least one characteristic peak of 2θ selected from 9.67° (±0.2°) and 19.63° (±0.2°) isshowed in the X-ray powder diffraction pattern of the solid form of the potassium salt of the compound of formula (I).

In some preferred embodiments, the solid form of the potassium salt of the compound of formula (I) has a structure as shown in formula (III), (F crystalline) 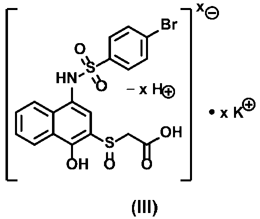 wherein x is 0.5-2.0. e.g. x is 0.5, 0.6, 0.7, 0.8, 0.9, 1.0, 1.1, 1.2, 1.3, 1.4, 1.5, 1.6, 1.7, 1.8, 1.9, or 2.0.

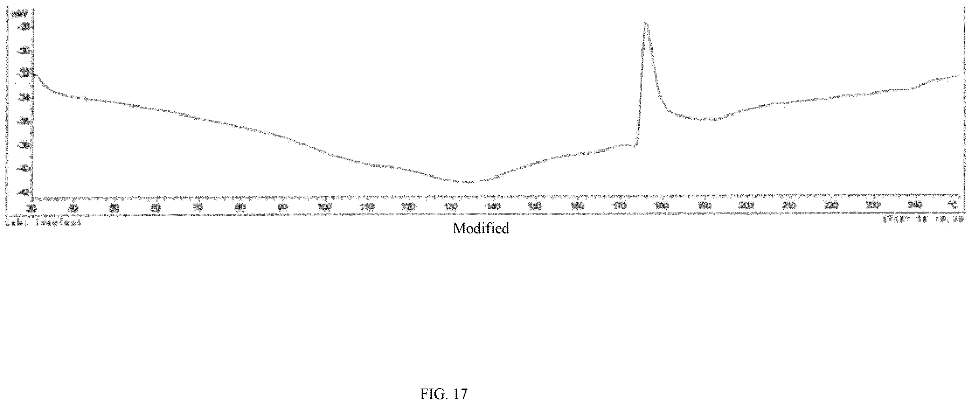

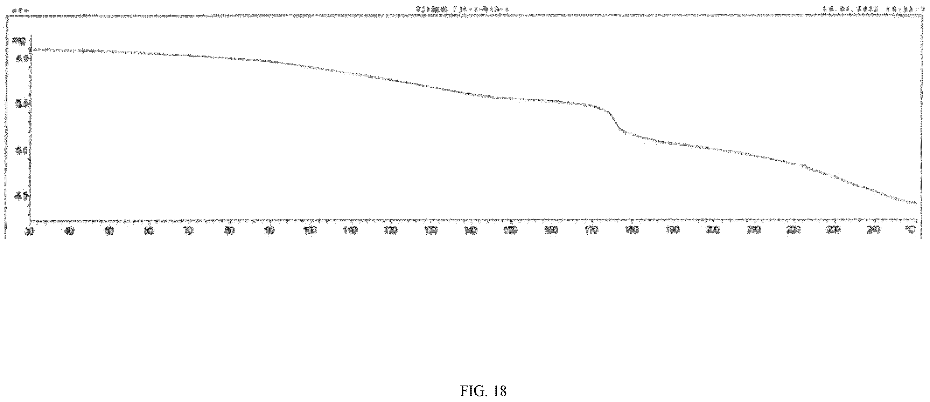

In some preferred embodiments, the solid form of the potassium salt of the compound of formula (I) has at least one of the following features:
the differential scanning calorimetry curve of the solid form of the potassium salt of the compound of formula (I) shows a differential scanning calorimetry curve pattern substantially identical to that of FIG. 17; and
the thermogravimetric analysis curve of the solid form of the potassium salt of the compound of formula (I) showing a thermogravimetric analysis curve pattern substantially identical to that of FIG. 18.

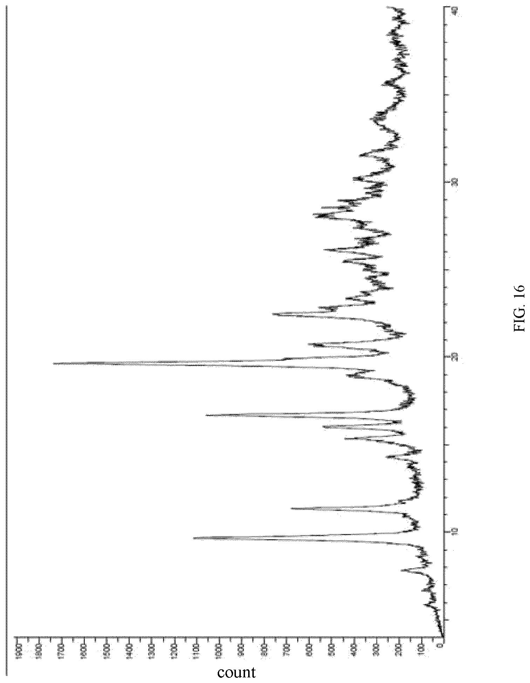

In some preferred embodiments, the solid form of the potassium salt of the compound of formula (I) shows an X-ray powder diffraction pattern substantially identical to that of FIG. 16.

In some preferred embodiments, at least one characteristic peak of 2θ selected from 9.67° (±0.2°), 11.34° (±0.2°), 16.68° (±0.2°), 19.63° (±0.2°) and 22.46° (±0.2°) is showed in the X-ray powder diffraction pattern of the solid form of the potassium salt of the compound of formula (I).

In some preferred embodiments, the solid form is a solid form of a calcium salt of the compound of formula (I), and at least one characteristic peak of 2θ selected from 11.53° (±0.2°) and 21.54° (±0.2°) is showed in the X-ray powder diffraction pattern of the solid form of the calcium salt of the compound of formula (I).

In some preferred embodiments, the solid form of the calcium salt of the compound of formula (I) has a structure as shown in formula (IV), (G crystal form) 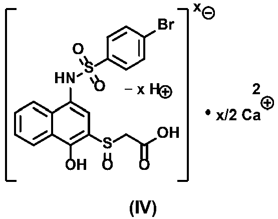 wherein x is 0.5-2.0. e.g. x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1. (G crystal form)

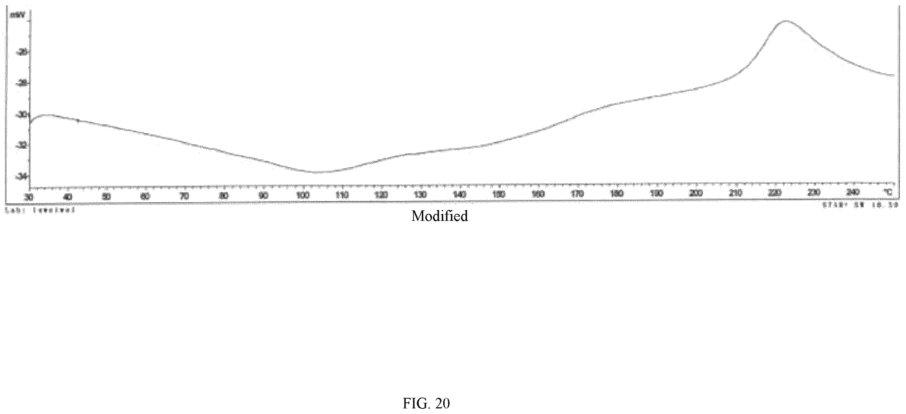

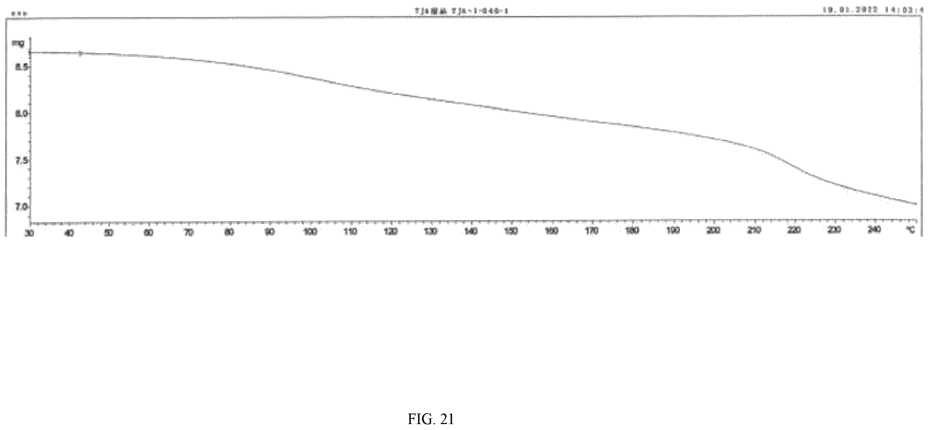

In some preferred embodiments, the solid form of the calcium salt of the compound of formula (I) has at least one of the following characteristics:
the differential scanning calorimetry curve of the solid form of the calcium salt of the compound of formula (I) shows a differential scanning calorimetry curve pattern substantially identical to that of FIG. 20; and
a thermogravimetric analysis curve of the solid form of the calcium salt of the compound of formula (I) shows a thermogravimetric analysis curve pattern substantially identical to that of FIG. 21.

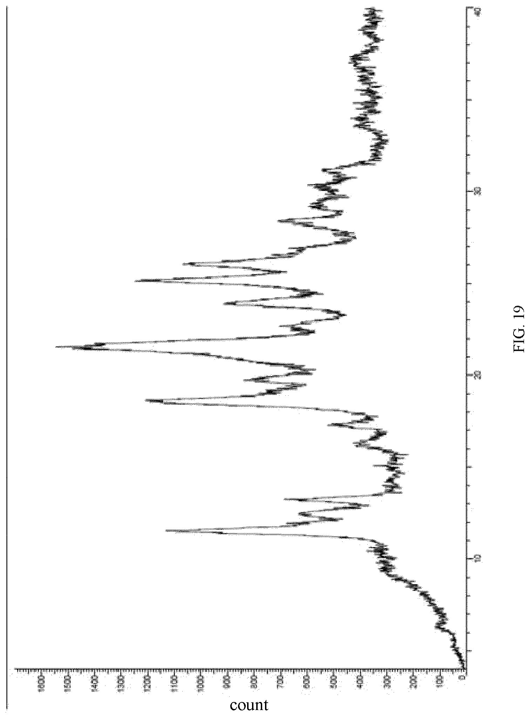

In some preferred embodiments, the solid form of the calcium salt of the compound of formula (I) shows an X-ray powder diffraction pattern substantially identical to FIG. 19.

In some preferred embodiments, at least one characteristic peak of 2θselected from 11.53° (±0.2°), 21.54° (±0.2°), 12.46° (±0.2°), 18.56° (±0.2°) , 20.92° (±0.2°), 25.20° (±0.2°) and 26.06° (±0.2°) is showed in the X-ray powder diffraction pattern of the solid form of the calcium salt of the compound of formula (I).

In some preferred embodiments, the solid form is a solid form of thetromethamine salt of the compound of formula (I), and at least one characteristic peak of 2θ selected from 8.94° (±0.2°) and 14 .35° (±0.2°) is showed in an X-ray powder diffraction pattern of the solid form of thetromethamine salt of the compound of formula (I).

In some preferred embodiments, the solid form of the tromethamine salt of the compound of formula (I) has a structure as shown in formula (V), (H crystal form) 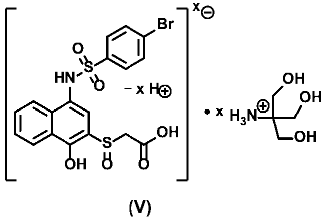 wherein x is 0.5-2.0. e.g., x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

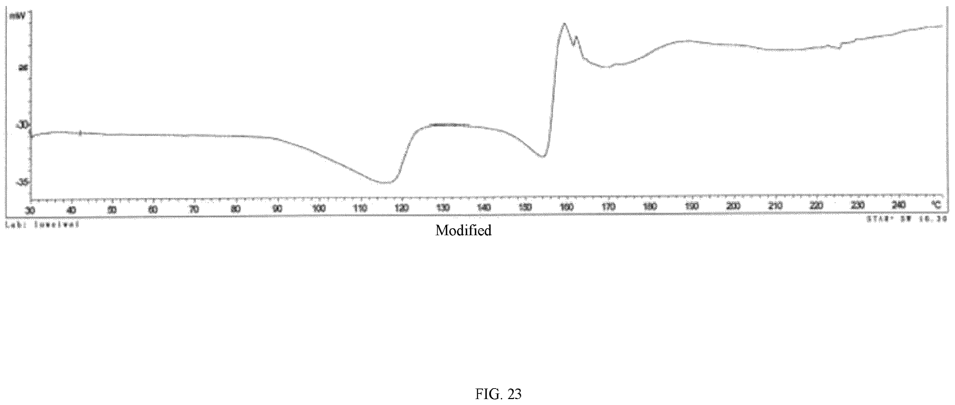

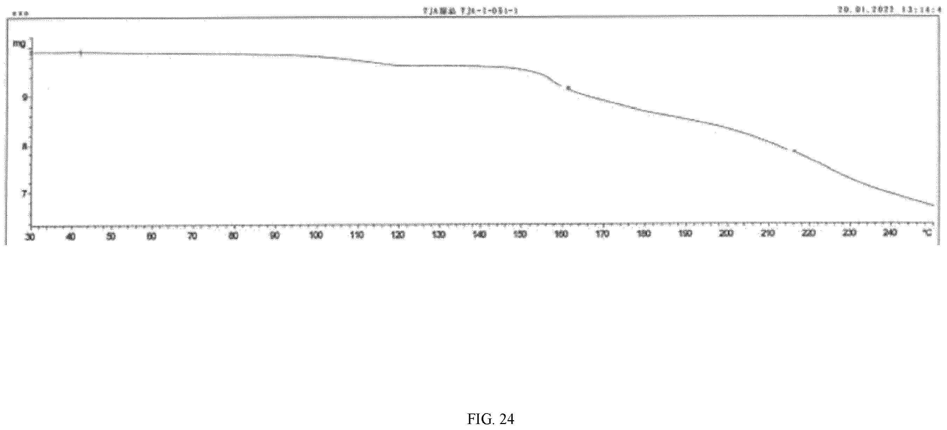

In some preferred embodiments, the solid form of the tromethamine salt of the compound of formula (I) has at least one of the following characteristics:
the differential scanning calorimetry curve of the solid form of thetromethamine salt of the compound of formula (I) shows a differential scanning calorimetry curve pattern substantially identical to that of FIG. 23; and
the thermogravimetric analysis curve of the solid form of the tromethamine salt of the compound of formula (I) shows a thermogravimetric analysis curve pattern substantially identical to that of FIG. 24.

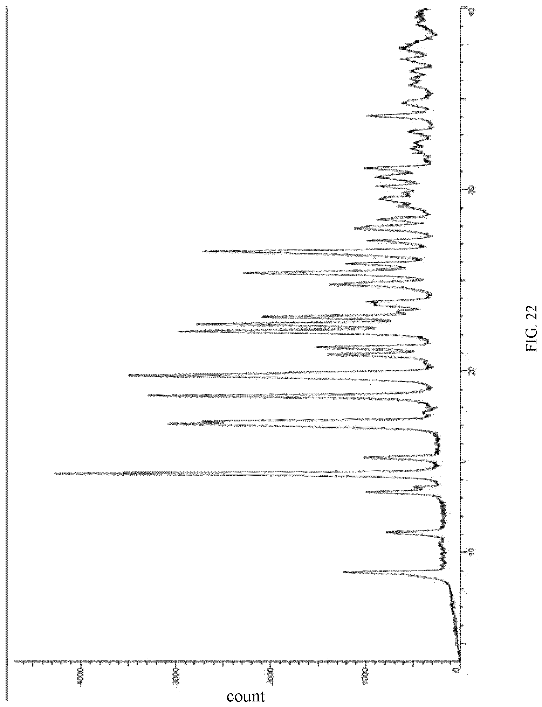

In some preferred embodiments, the solid form of thetromethamine salt of the compound of formula (I) shows an X-ray powder diffraction pattern substantially identical to that of FIG. 22.

In some preferred embodiments, at least one characteristic peak of 2θ selected from 8.94° (±0.2°), 14 .35° (±0.2°), 17.09° (±0.2°), 18.69° (±0.2°), 19.76° (±0.2°), 22.19° (±0.2° ), 22.59° (±0.2°), 22.99° (±0.2°), 25.40° (±0.2°), and 26.58° (±0.2°) is showed in the X-ray powder diffraction pattern of the solid form of the tromethamine salt of the compound of formula (I).

In some preferred embodiments, the solid form is a solid form of a lysine salt of the compound of formula (I), and at least one characteristic peak of 2θ selected from 12.31° (±0.2°) and 19.66° (±0.2°) is showed in the X-ray powder diffraction pattern of the solid form of the lysine salt of the compound of formula (I).

In some preferred embodiments, the solid form of the lysine salt of the compound of formula (I) has a structure as shown in formula (VI), (I crystal form) 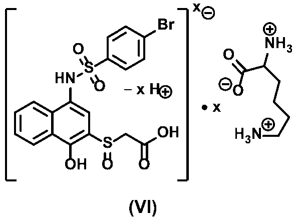 wherein x is 0.5-2.0. e.g., x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

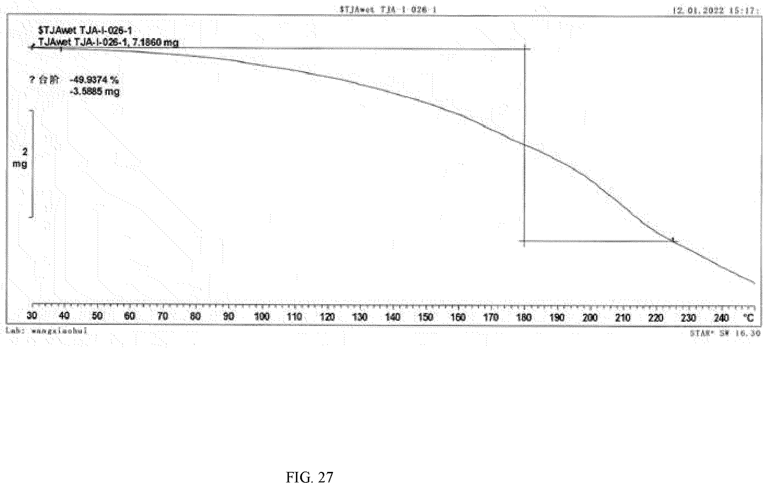

In some preferred embodiments, the solid form of the lysine salt of the compound of formula (I) has at least one of the following features:
the thermogravimetric analysis curve of the solid form of the lysine salt of the compound of formula (I) shows a thermogravimetric analysis curve pattern substantially identical to that of Fig.27.

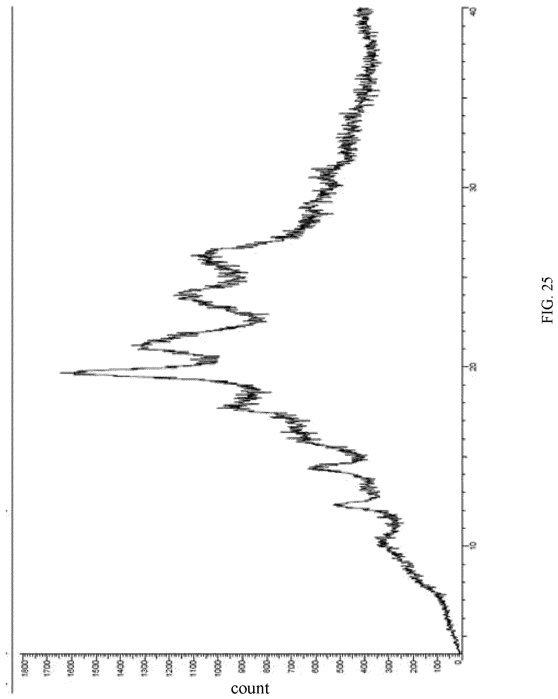

In some preferred embodiments, the solid form of the lysine salt of the compound of formula (I) shows an X-ray powder diffraction pattern substantially identical to that of FIG.25.

In some preferred embodiments, at least one characteristic peak of 2θ selected from 12.31° (±0.2°), 17.72° (±0.2°), 21.16° (±0.2°), 24.13° (±0.2°), and 19.66° (±0.2°) is showed in the X-ray powder diffraction pattern of the solid form of the lysine salt of the compound of formula (I).

In some preferred embodiments, the solid form is a solid form of a tert-butylamine salt of the compound of formula (I), and at least one characteristic peak) of 2θ selected from 7.15° (±0.2°) and 10.14° (±0.2° is showed in the X-ray powder diffraction pattern of the solid form of a tert-butylamine salt of the compound of formula (I).

In some preferred embodiments, the solid form of the tert-butylamine salt of the compound of formula (I) has a structure as shown in formula (VII), (J crystal form) 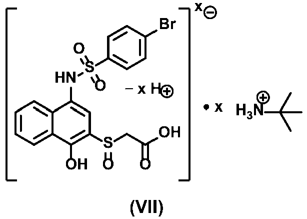 wherein x is 0.5-2.0. e.g., x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

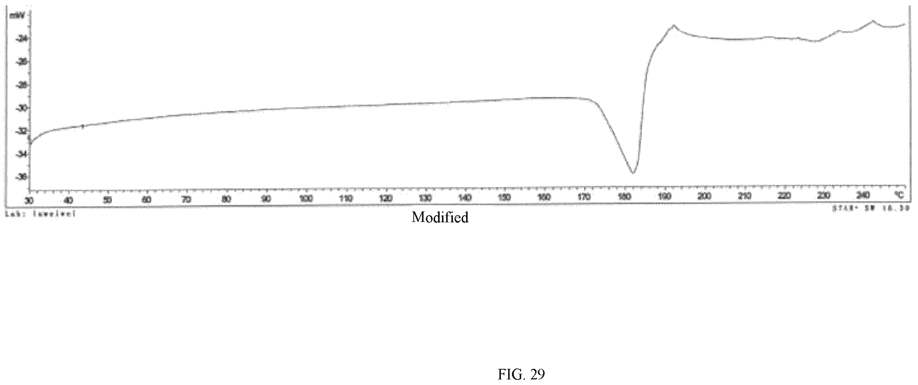

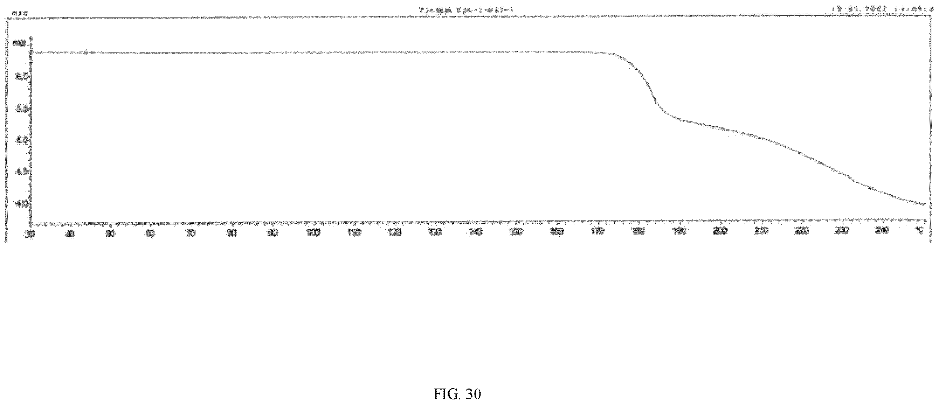

In some preferred embodiments, the solid form of the tert-butylamine salt of a compound of formula (I) has at least one of the following features:
the differential scanning calorimetry curve of the solid form of the tert-butylamine salt of a compound of formula (I) shows a differential scanning calorimetry curve pattern substantially identical to that of FIG. 29; and
the thermogravimetric analysis curve of the solid form of the tert-butylamine salt of the compound of formula (I) shows a thermogravimetric analysis curve pattern substantially identical to that of FIG. 30.

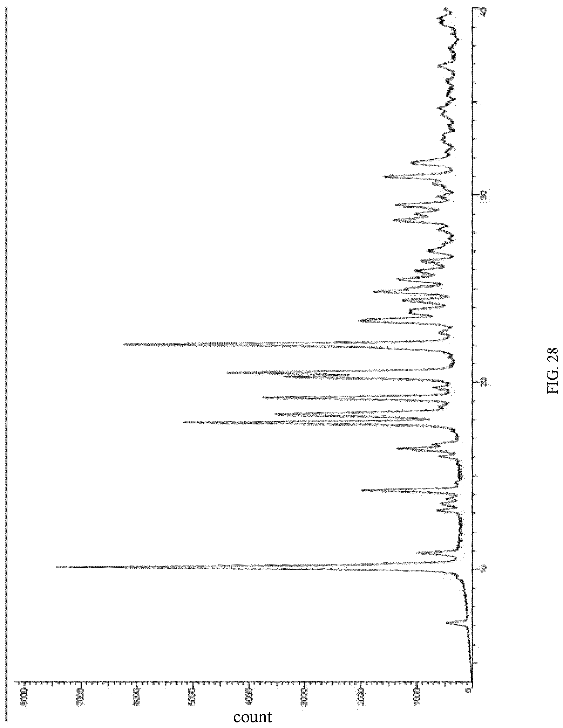

In some preferred embodiments, the solid form of the tert-butylamine salt of the compound of formula (I) shows an X-ray powder diffraction pattern substantially identical to FIG. 28.

In some preferred embodiments, at least one characteristic peak of 2θ selected from the group consisting of 7.15° (±0.2°), 17.85° (±0.2°), 18.28° (±0.2°), 19.19° (±0.2°), 20.51° (±0.2°), 22.03° (±0.2°) and 10.14° (±0.2°) is showed in the X-ray powder diffraction pattern of the solid form of the tert-butylamine salt of the compound of formula (I).

In some preferred embodiments, the solid form is a solid form of a diisopropylamine salt of the compound of formula (I), and at least one characteristic peak of 2θ selected from 8.87° (±0.2°) and 17.20° (±0.2°) is showed in the X-ray powder diffraction pattern of the solid form of a diisopropylamine salt of a compound of formula (I).

In some preferred embodiments, the solid form of the diisopropylamine salt of the compound of formula (I) has a structure as shown in formula VIII), (K crystal form) 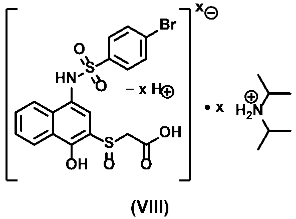 wherein x is 0.5-2.0. e.g., x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

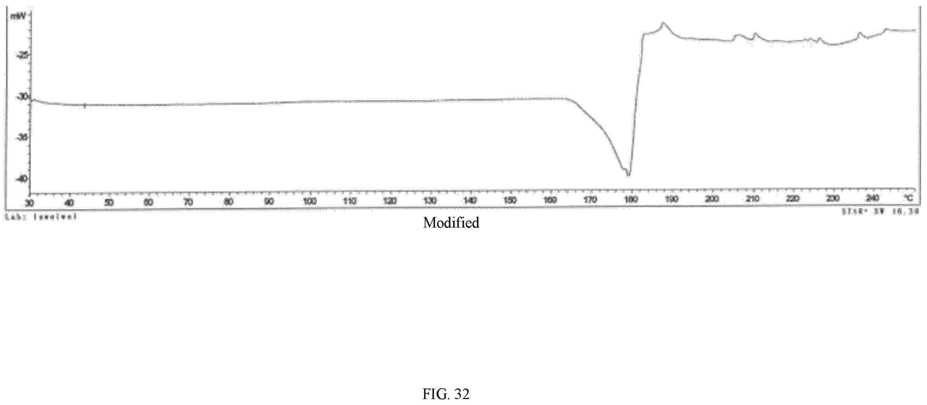

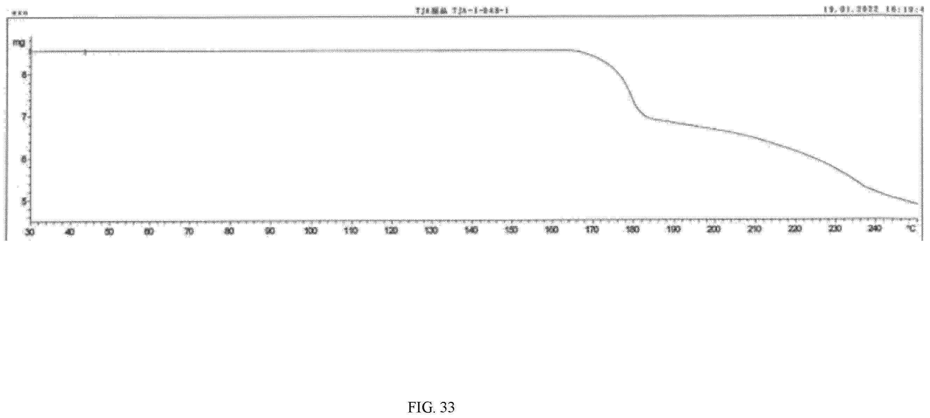

In some preferred embodiments, the solid form of the diisopropylamine salt of the compound of formula (I) has at least one of the following features:
the differential scanning calorimetry curve of the solid form of the diisopropylamine salt of a compound of formula (I) shows a differential scanning calorimetry curve pattern substantially identical to that of FIG. 32; and
the thermogravimetric analysis curve of the solid form of the diisopropylamine salt of the compound of formula (I) shows a thermogravimetric analysis curve pattern substantially identical to that of FIG. 33.

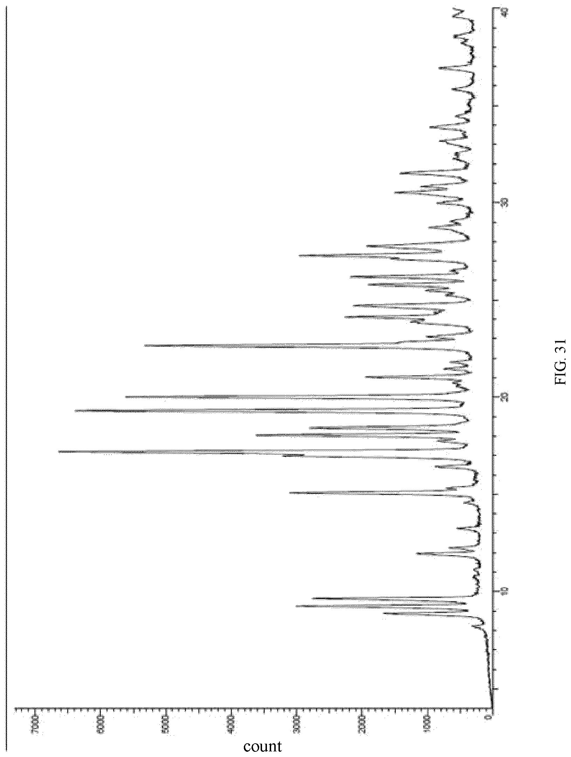

In some preferred embodiments, the solid form of the diisopropylamine salt of a compound of formula (I) shows an X-ray powder diffraction pattern substantially identical to that of FIG. 31.

In some preferred embodiments, at least one characteristic peak of 2θselected from 8.87° (:f:0.2°), 9.25° (±0.2°), 9.65° (±0.2°), 15.07° (±0.2°), 16.97° (±0.2°), 18.06° (±0.2°), 19.31° (±0.2°), 20.01° (±0.2°), 22.65° (±0.2°), 27.29° (±0.2°), and 17.20° (±0.2°) is showedinan X-ray powder diffraction pattern of the solid form of the diisopropylamine salt of the compound of formula (I).

In some preferred embodiments, the solid form is a solid form of an ethanolamine salt of the compound of formula (I), and at least one characteristic peak of 2θ selected from 7.62° (±0.2°) and 19.70° (±0.2°) is showed in the X-ray powder diffraction pattern of the solid form of an ethanolamine salt of a compound of formula (I).

In some preferred embodiments, the solid form of the ethanolamine salt of the compound of formula (I) has a structure as shown in formula (IX), (L crystal form) 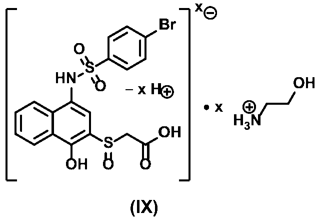 wherein x is 0.5-2.0. e.g., x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

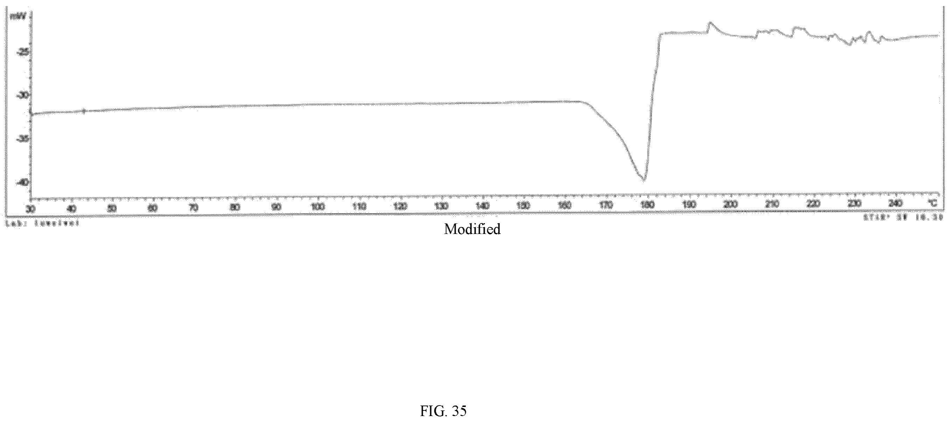

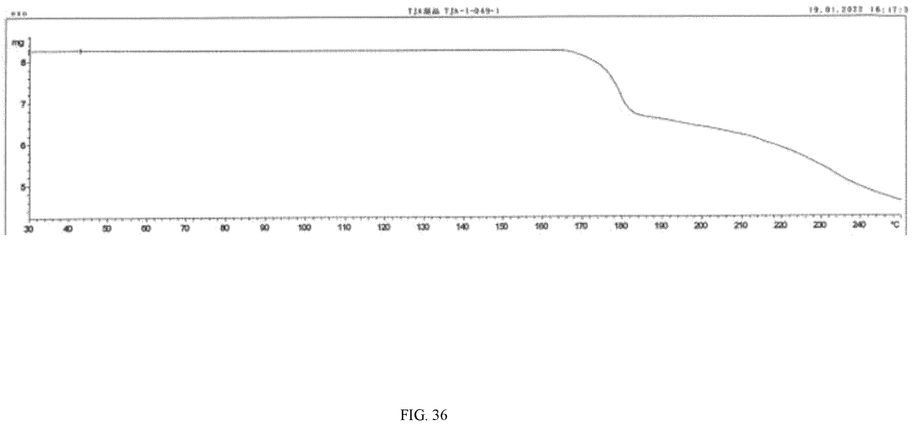

In some preferred embodiments, the solid form of the ethanolamine salt of the compound of formula (I) has at least one of the following features:
the differential scanning calorimetry curve of the solid form of the ethanolamine salt of the compound of formula (I) shows a differential scanning calorimetry curve pattern substantially identical to that of FIG. 35; and
the thermogravimetric analysis curve of the solid form of the ethanolamine salt of the compound of formula (I) shows a thermogravimetric analysis curve pattern substantially identical to that of FIG. 36.

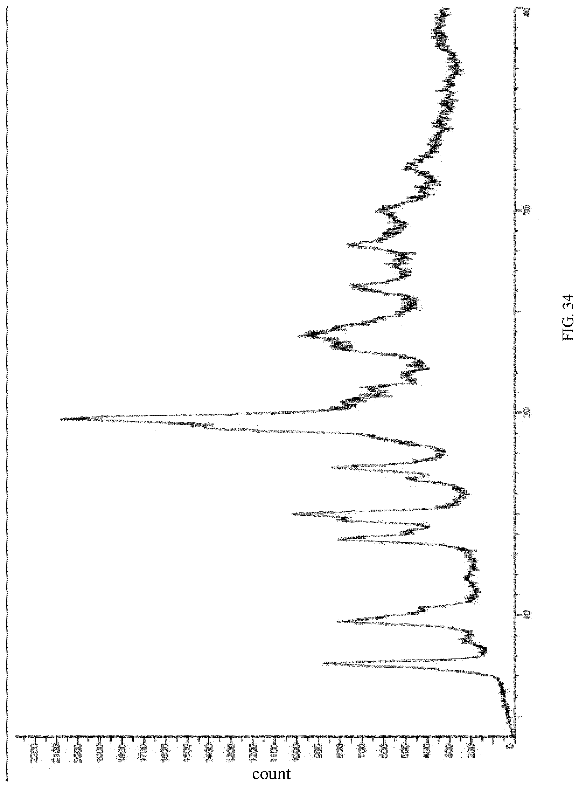

In some preferred embodiments, the solid form of the ethanolamine salt of the compound of formula (I) shows an X-ray powder diffraction pattern substantially identical to FIG. 34.
In some preferred embodiments, at least one characteristic peak of 2θ selected from 7.62° (±0.2°), 9.72° (±0.2°), 14.98° (±0.2°), 19.34° (±0.2°) and 19.70° (±0.2°) is showed in the X-ray powder diffraction pattern of the solid form of the ethanolamine salt of the compound of formula (I).

In some preferred embodiments, the solid form is a solid form of a diethanolamine salt of the compound of formula (I), and at least one characteristic peak of 2θ selected from 6.33° (±0.2°) and 19.87° (±0.2°) is showed in the X-ray powder diffraction pattern of the solid form of the diethanolamine salt of the compound of formula (I).

In some preferred embodiments, the solid form of the diethanolamine salt of the compound of formula (I) has a structure as shown in formula (X), (M crystalline) 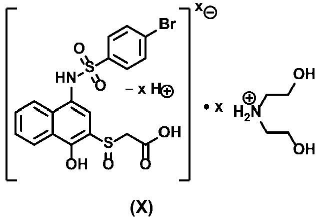 wherein x is 0.5-2.0. e.g., x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

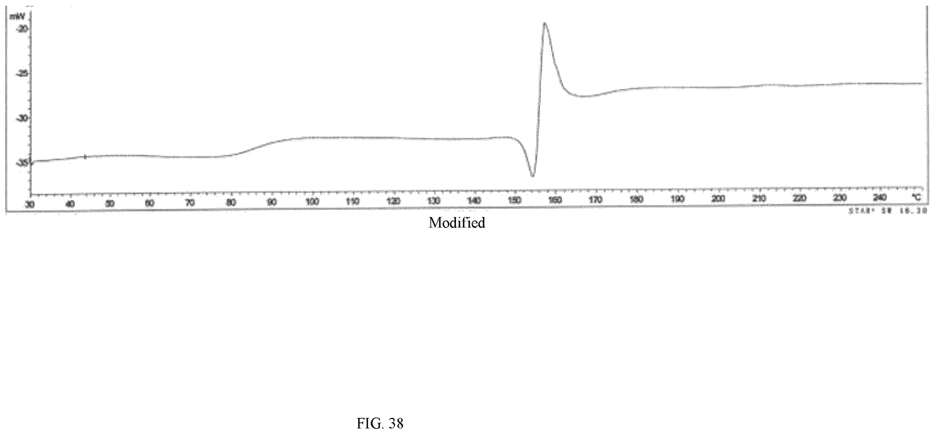

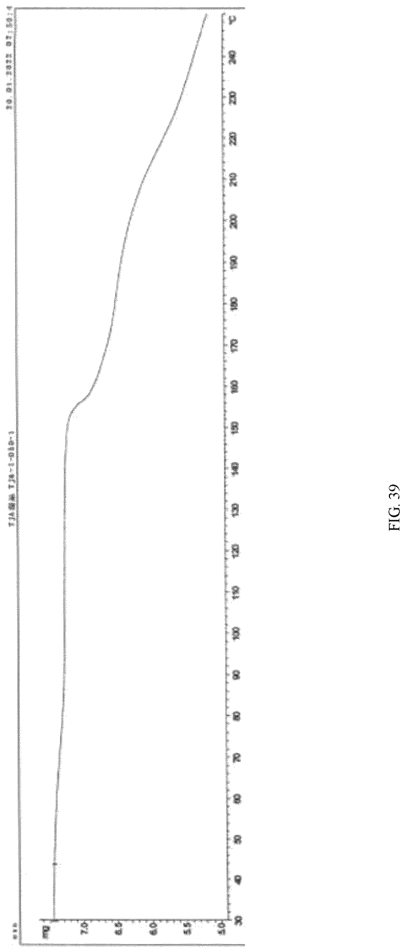

In some preferred embodiments, the solid form of the diethanolamine salt of the compound of formula (I) has at least one of the following features:
the differential scanning calorimetry curve of the solid form of the diethanolamine salt of the compound of formula (I) shows a differential scanning calorimetry curve pattern substantially identical to that of FIG. 38; and
the thermogravimetric analysis curve of the solid form of the diethanolamine salt of the compound of formula (I) shows a thermogravimetric analysis curve pattern substantially identical to that of FIG. 39.

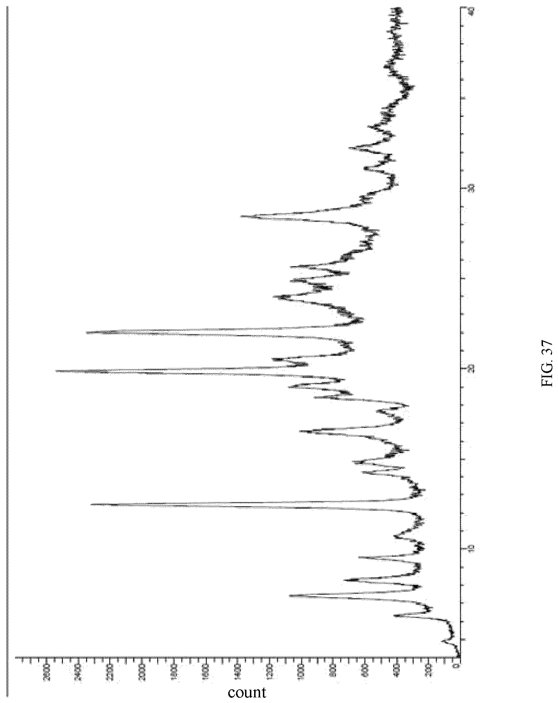

In some preferred embodiments, the solid form of the diethanolamine salt of a compound of formula (I) shows an X-ray powder diffraction pattern substantially identical to that of FIG. 37.

In some preferred embodiments, at least onecharacteristic peak of 2θselected from 6.33° (±0.2°), 7.41° (±0.2°), 12.46° (±0.2°), 22.02° (±0.2°), 28.45° (±0.2°) and 19.87° (±0.2°) is showed in the X-ray powder diffraction pattern of the solid form of the diethanolamine salt of the compound of formula (I).

In a second aspect of the present invention, there is provided a solid form (E crystal form) of a free acid of a compound of formula (I); at least one characteristic peak of 2θ selected from 8.92° (±0.2°) and 23.31° (±0.2°) is showed in the X-ray powder diffraction pattern of the solid form of the free acid of the compound of formula (I).

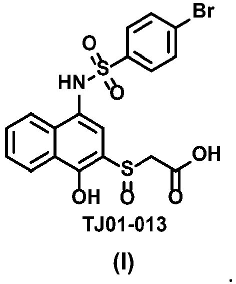

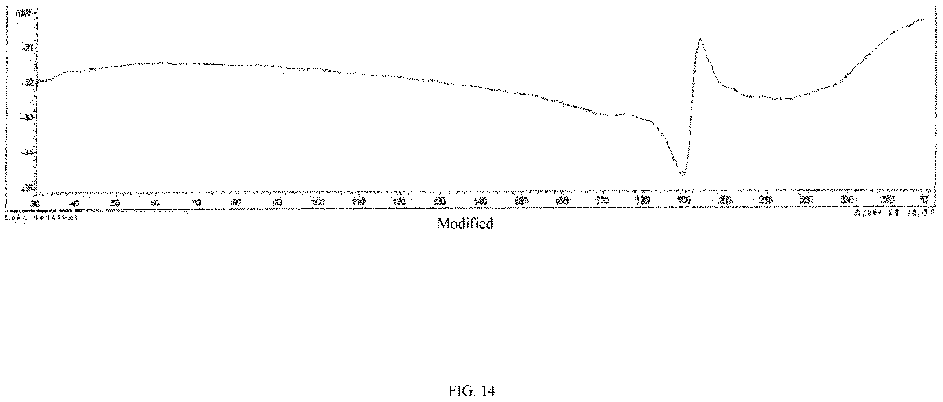

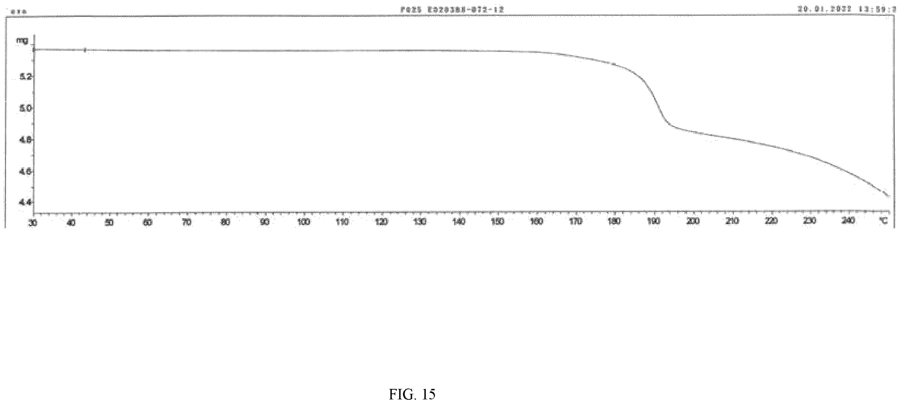

In some preferred embodiments, the solid form of the free acid of the compound of formula (I) further has at least one of the following features:
the differential scanning calorimetry curve of the solid form of the ethanolamine salt of the compound of formula (I) shows a differential scanning calorimetry curve pattern substantially identical to that of FIG. 14; and
the thermogravimetric analysis curve of the solid form of the ethanolamine salt of the compound of formula (I) shows a thermogravimetric analysis curve pattern substantially identical to that of FIG. 15.

In some preferred embodiments, at least one characteristic peak of 2θselected from 8.92° (±0.2°), 23.31° (±0.2°), 27.41° (±0.2°), 19.70° (±0.2°), 16.51° (±0.2°), 12.27° (±0.2°), 21.50° (±0.2°), 18.23° (±0.2°) and 18.37° (±0.2°) is showed in the X-ray powder diffraction pattern of the solid form of the free acid of the compound of formula (I).

In a third aspect of the present invention, there is provided a pharmaceutical composition, which comprises the solid form of the compound of formula (I) as described in any one of first to second aspectsof the present invention, and a pharmaceutically acceptable carrier or excipient.

In the fourth aspect of the present invention, there is provided a method of preparing the solid form of the sodium salt of the compound of formula (I), the method comprising the steps: dissolving the compound of formula (I) in a reaction medium, and adding a sodium-containing base to react to obtain the compound.

In some preferred embodiments, the reaction medium isselectedfromat least one of the compounds, including methanol, ethanol, isopropanol, tert-butanol, acetone, acetonitrile and ethyl acetate, or a mixture containing water and at least one of the coumponds, including methanol, ethanol, isopropanol, tert-butanol, acetone, acetonitrile and ethyl acetate, for examples, acetone, a mixture of acetone and water, or a mixture of acetonitrile and water.

In some preferred embodiments, the sodium-containing base is selected from at least one of compounds, including sodium bicarbonate, sodium carbonate, sodium hydroxide, sodium acetate, sodium formate, sodium methoxide, sodium ethoxideethanol and sodium tert- butoxide.

In some preferred embodiments, the molar ratio of the compound of formula (I) and the one with sodium-containing base in the reaction system is 1: (0.9-1.1).

In some preferred embodiments, dissolving the compound of formula (I) in acetone at 35-55°C (preferably 40-50°C), and adding a sodium bicarbonate solution, afterthe solid is precipitated, keeping warm and standing still for at least 20 minutes (preferably for at least 30 minutes), and then cooling to room temperature to obtain the solid form of the sodium salt of the compound of formula (I).

In some preferred embodiments, dissolving the compound of formula (I) in acetone at 35-55°C, cooling to room temperature, adding a sodium bicarbonate solution and stirring, afterthe solid is precipitated, keeping warm and standing still for at least 20 minutes (preferably for at least 30 minutes), to obtain the solid form of the sodium salt of the compound of formula (I).

In some preferred embodiments, dissolving the compound of formula (I) in acetone at 35-55°C, adding the sodium bicarbonate solution and stirring, afterthe solid is precipitated, keeping warm and stirring for at least 20 minutes (preferably for at least 30 minutes), and then cooling to room temperature to obtain the solid form of the sodium salt of the compound of formula (I).

In some preferred embodiments, dissolving the compound of formula (I) in acetone at 35-55°C, adding a methanol solution of sodium methoxide and stirring, after the solid is precipitated, keeping warm and stirring for at least 50 minutes (preferably for at least 60 minutes), and then cooling to room temperature and stirring for at least 50 minutes (preferably for at least 60 minutes), to obtain the solid form of the sodium salt of the compound of formula (I).

In some preferred embodiments, dissolving the compound of formula (I) in acetone at 35-55°C, adding the sodium bicarbonate solution and stirring, and afterthe solid is precipitated, standing still for at least 50 minutes (preferably for at least 60 minutes), to btain the solid form of the sodium salt of the compound of formula (I).

In the fifth aspect of the present invention, there is provided uses of the solid form of the compound of formula (I) as described in the first aspect of the present invention or the second aspect of the present invention or the pharmaceutical composition as described in the third aspect of the present invention for (i) preparing a drug for preventing and/or treating diseases associated with kidney injury; and/or
(ii) preventing and/or treating diseases associated with kidney injury; and/or
(iii) preventing and/or treating diseases associated with mitochondrial dysfunction; and/or
(iv) preparing a drug for preventing and/or treating diseases associated with mitochondrial dysfunction.

The present invention has at least the following advantages over the prior art:
(1) The present inventionprepares and obtains various solid forms of the compounds of formula (I), such as free acids, sodium salts, potassium salts, calcium salts, tromethamine salts, lysine salts, tert-butylammonium salts, diisopropylammonium salts, ethanolammonium salts, diethanolamine salts of the compounds of formula (I), and provides corresponding methods of identifying;
(2) The solid forms of the sodium salt of the compound of formula (I) prepared in the preferred embodiment of the present invention is excellent in terms of crystalline stability, biological activity, safety, bioavailability, etc., in addition to low hygroscopicity, good water solubility, and good prospects for pharmaceutical development.

It should be understood that, within the scope of the present invention, each of the above technical features of the present invention and each of the technical features specifically described hereinafter (e.g., embodiments) can be combined with each other, thereby constituting a new or preferred technical solution. For the sake of space, we will not repeat them herein.

### Description of the drawings

One or more embodiments are illustrated exemplarily by means of pictures in the corresponding accompanying drawings, which exemplary illustrations do not constitute a limitation of the embodiments.
FIG. 1 is an X-ray powder diffraction pattern of an A crystal form according to an embodiment of the present invention;
FIG. 2 is a differential scanning calorimetry pattern of an A crystal form according to an embodiment of the present invention;
FIG. 3 is a thermogravimetric analysis curve of an A crystal form according to an embodiment of the present invention;
FIG. 4 is an X-ray powder diffraction pattern of a B crystal form according to an embodiment of the present invention;
FIG. 5 is a differential scanning calorimetry curve pattern of a B crystal form according to an embodiment of the present invention;
FIG. 6 is a thermogravimetric analysis curve pattern of a B crystal form according to an embodiment of the present invention;
FIG. 7 is an X-ray powder diffraction pattern of a C crystal formaccording to an embodiment of the present invention;
FIG. 8 is a differential scanning calorimetry curve pattern of a C crystal formaccording to an embodiment of the present invention;
FIG. 9 is a thermogravimetric analysis curve pattern of a C crystal form according to an embodiment of the present invention;
FIG. 10 is an X-ray powder diffraction pattern of a D crystal form according to an embodiment of the present invention;
FIG. 11 is a differential scanning calorimetry curve pattern of a D crystal form according to an embodiment of the present invention;
FIG. 12 is a thermogravimetric analysis curve pattern of a D crystal form according to an embodiment of the present invention;
FIG. 13 is an X-ray powder diffraction pattern of an E crystal form according to an embodiment of the present invention;
FIG. 14 is a differential scanning calorimetry curve pattern of an E crystal form according to an embodiment of the present invention;
FIG. 15 is a thermogravimetric analysis curve pattern of an E crystal form according to an embodiment of the present invention;
FIG. 16 is an X-ray powder diffraction pattern of aFcrystal form according to an embodiment of the present invention;
FIG. 17 is a differential scanning calorimetry curve patternof aFcrystal form according to an embodiment of the present invention;
FIG. 18 is aa thermogravimetric analysis curve patternof a Fcrystal form according to an embodiment of the present invention;
FIG. 19 is an X-ray powder diffraction pattern of a G crystal form according to an embodiment of the present invention;
FIG. 20 is a differential scanning calorimetry curve pattern of a G crystal form according to an embodiment of the present invention;
FIG. 21 is a thermogravimetric analysis curve pattern of a G crystal form according to an embodiment of the present invention;
FIG. 22 is an X-ray powder diffraction pattern of an H crystal form according to an embodiment of the present invention;
FIG. 23 is a differential scanning calorimetry curve pattern of an H crystal form according to an embodiment of the present invention;
FIG. 24 is a thermogravimetric analysis curve pattern of an H crystal form according to an embodiment of the present invention;
FIG. 25 is an X-ray powder diffraction pattern of an Icrystal form according to an embodiment of the present invention;
FIG. 26 is a diagram of the mutual conversion relationship among multiple crystal forms of sodium salt according to an embodiment of the present invention;
FIG. 27 is a thermogravimetric analysis curve pattern of an Icrystal form according to an embodiment of the present invention;
FIG. 28 is an X-ray powder diffraction pattern of a Jcrystal form according to an embodiment of the present invention;
FIG. 29 is a differential scanning calorimetry curvepattern of a Jcrystal form according to an embodiment of the present invention;
FIG. 30 is a thermogravimetric analysis curve pattern of a Jcrystal form according to an embodiment of the present invention;
FIG. 31 is an X-ray powder diffraction pattern of a K crystal form according to an embodiment of the present invention;
FIG. 32 is a differential scanning calorimetry curvepattern of a K crystal form according to an embodiment of the present invention;
FIG. 33 is a thermogravimetric analysis curve pattern of a K crystal form according to an embodiment of the present invention;
FIG. 34 is an X-ray powder diffraction pattern of an L crystal form according to an embodiment of the present invention;
FIG. 35 is a differential scanning calorimetry curvepattern of an L crystal form according to an embodiment of the present invention;
FIG. 36 is a thermogravimetric analysis curve pattern of an L crystal form according to an embodiment of the present invention;
FIG. 37 is an X-ray powder diffraction pattern of an M crystal form according to an embodiment of the present invention;
FIG. 38 is a differential scanning calorimetry curve pattern of an M crystal form according to an embodiment of the present invention;
FIG. 39 is a thermogravimetric analysis curve pattern of an M crystal form according to an embodiment of the present invention;
FIG. 40 is a DVSpattern of a sample of D crystal form according to an embodiment of the present invention;
FIG. 41 is a solubility graph in FaSSIF buffer, FeSSIF buffer, water and SGF of the D crystal form according to an embodiment of the present invention, with three columns denoting 0.5 hours, 2 hours and 24 hours from left to right;
FIG. 42 is a thermogram of the level of mitophagy induced by different concentrations of the D crystal form according to an embodiment of the present invention;
FIG. 43 is a graph of changes in serum creatinine before and after administration of the D crystal form in a model of renal injury caused by ischemia and reperfusion in a rat unilateral kidney according to an embodiment of the present invention, with five columns denoting the sham group, the modelcontrol group, the TJ0113 3 mg/kg group, the TJ0113 10 mg/kg group, and the TJ0113 30 mg/kg group from left to right;
FIG. 44 is a graph of changes in blood urea nitrogen before and after administration of the D crystal form in a model of renal injury caused by ischemia and reperfusion in a unilateral kidney in a rat according to an embodiment of the present invention, with five columns denoting the sham group, the model control group, the TJ0113 3 mg/kg group, the TJ0113 10 mg/kg group, and the TJ0113 30 mg/kg group;
FIG. 45 is a graph of changes in serum creatinine clearance rate before and after administration of the D crystal form in a model of renal injury caused by ischemia and reperfusion in a unilateral kidney in a rat according to an embodiment of the present invention, with five columns denoting the sham group, the modelcontrol group, the TJ0113 3 mg/kg group, the TJ0113 10 mg/kg group, and the TJ0113 30 mg/kg groupfrom left to right;
FIG. 46 is a graph of Caspase9 changes in renal tissue before and after administration of the D crystal form in a model of renal injury caused by ischemia and reperfusion in a unilateral kidney in a rat in accordance with an embodiment of the present invention, with fivecolumnsdenoting the sham group, the modelcontrol group, the TJ0113 3 mg/kg group, the TJ0113 10 mg/kg group, and the TJ0113 30 mg/kg groupfrom left to right;
FIG. 47 is a graph of changes in renal tissue IL-6 before and after administration of the D crystal form in a model of renal injury caused by ischemia and reperfusion in a rat unilateral kidney in accordance with an embodiment of the present invention, with five columns denoting the sham group, the modelcontrol group, the TJ0113 3 mg/kg group, the TJ0113 10 mg/kg group, and the TJ0113 30 mg/kg group from left to right;
FIG. 48 is a graph of changes in renal tissue IL-1β before and after administration of the D crystal form in a model of renal injury caused by ischemia and reperfusion in a rat unilateral kidney in accordance with an embodiment of the present invention, with five columns denoting the sham group, the modelcontrol group, the TJ0113 3 mg/kg group, the TJ0113 10 mg/kg group, and the TJ0113 30 mg/kg group from left to right;
FIG. 49 is a graph of TUNEL changes in renal tissue before and after administration of the D crystal form in a model of renal injury caused by ischemia and reperfusion in a unilateral kidney in a rat according to an embodiment of the present invention, with 5 columns from left to right being the sham group, themodelcontrol group, the TJ0113 3 mg/kg group, the TJ0113 10 mg/kg group, and the TJ0113 30 mg/kg group.

### Detailed description of embodiments

The present invention prepares and obtains various solid forms of compound TJ01-013 (as shown in formula I) and researches on the stability, solubility, safety and bioavailability of each crystal form, and finally finds that the D crystal form of the sodium salt of compound TJ01-013 has the best prospects for pharmaceutical development.

### Solid form of compound TJ01-013

Various solid forms of compound TJ01-013 (as shown in formula I) are involved in the present invention, including: a sodium salt of the compound of formula (I), a potassium salt of the compound of formula (I), a calcium salt of the compound of formula (I), atromethamine salt of the compound of formula (I), a lysine salt of the compound of formula (I), a tert-butyl amine salt of the compound of formula (I), adiisopropyl amine salt of the compound of formula (I), a ethanolamine salt of the compound of formula (I), and a diethanolamine salt of the compound of formula (I);

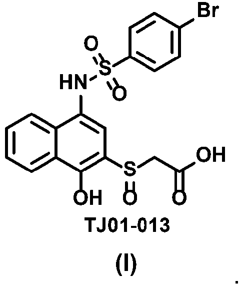

In the present invention, the term "solid form" refers to a type of solid material including amorphous as well as crystal forms. The term "crystal form" refers to polycrystal forms as well as solvent compounds, hydrates and the like. The term "polycrystalline" refers to a particular crystalline structure having specific physical properties (e.g., X-ray scattering, melting point, and the like).

In the present invention, the above-mentioned solid forms are characterized by methods conventional in the art, such as X-ray powder diffraction, differential scanning calorimetry and thermogravimetric analysis. It should be understood that the positions and relative intensities of the peaks in the X-ray powder diffractograms may be slightly shifted due to various factors known to the skilled person. For example, shifts in the position of the peaks of the plots or the relative intensity of the peaks may occur due to the equipment used, the source of the rays, and the method and length of data collection. However, the skilled person will be able to compare the X-ray powder diffraction patterns shown in the accompanying drawings herein with those with an unknown solid form to confirm the identity of the solid form. As used in the present invention, the terms "substantially", "generally", "essentially" mean that there is an uncertainty in the measurement of the 2θ degree of ±0.3 (expressed as 2θ degree), preferably ±0.2 (expressed as 2θ degrees), or an uncertainty in the measurement of ±0.3°C when applied to a DSC curve, or a ±2% variation in weight loss when applied to a TGA thermal analysis pattern.

### (1) Sodium salt of a compound of formula (I)

In the present invention, the sodium salt of the compound of formula (I) refers to the salt obtained by contact reaction of the compound of formula (I) with a sodium-containing base (the sodium-containing base includingaorganic basecontainingsodium, for example, sodium ethoxide; inorganic basecontainingsodium, for example, sodium hydroxide; and a strong-base-weak-acidsalt containingsodium, for example, sodium bicarbonate, sodium dihydrogen carbonate, and the like). The sodium salt of the compound of formula (I) preferably has the structure shown in formula (II) below, with x selected from 0 .5-2, such as 0.5, 0.6, 0.7, 0.8, 0.9, 1.0, 1.1, 1.2, 1.3, 1.4, 1.5, 1.6, 1.7, 1.8, 1.9, or 2.0.

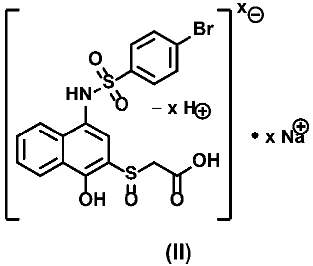

Depending on the method of preparation, at least four different crystal forms (crystals A-D) of the sodium salt of the compound of formula (I) are obtained in the present invention.

In some embodiments, the solid form of the sodium salt of the compound of formula (I) is prepared by the following method, comprising the steps:
dissolving the compound of formula (I) in acetone at 40-50°C, and adding a base containing sodium and reacting to obtain it.

In some embodiments, dissolving the compound of formula (I) in acetone at 40-50°C, and adding a sodium bicarbonate solution, after the solid is precipitated, keeping warm and standing still for at least 30 minutes, and thencooling to room temperature and leaching to obtain the solid form of the sodium salt of the compound of formula (I) (crystal form A).

In some embodiments, dissolving the compound of formula (I) in acetone at 40-50°C, cooling to room temperature, adding a sodium bicarbonate solution and stirring, after the solid is precipitated, standing still for at least 30 minutes to obtain the solid form of the sodium salt of the compound of formula (I) (crystal form B).

In some embodiments, dissolving the compound of formula (I) in acetone at 40-50°C, adding the sodium bicarbonate solution and stirring, after the solid is precipitated, keeping warm and stirring for at least 30 minutes, and then cooling to room temperature and leaching to obtain the solid form of the sodium salt of the compound of formula (I) (crystal form C).

In some preferred embodiments, dissolving the compound of formula (I) in acetone at 40-50°C, adding the sodium methoxide solution in methanol and stirring, after the solid is precipitated, keeping warm and standing still for at least 60 minutes, and then cooling to room temperature and stirring for at least 60 minutes, and leaching to obtain the solid form of the sodium salt of the compound of formula (I) (crystal form D).

In some preferred embodiments, dissolving the compound of formula (I) in acetone at 40-50°C, adding the sodium bicarbonate solution and stirring, after the solid is precipitated, keeping warm and standing still for at least 60 minutes, and leaching to obtain the solid formof the sodium salt of the compound of formula (I) (crystal form D).

As a solid form (crystal form D) of the sodium salt of the compound of formula (I), the X-ray powder diffraction pattern thereof at least has the following features: the X-ray powder diffraction pattern shows characteristic peaks of 2θ of 7.06° (±0.2°) and 20.87° (±0.2°); more preferably, the X-ray powder diffraction pattern shows characteristic peaks of 2θ of 7.06° (±0.2°) and 20.87° (±0.2 °), and at least one characteristic peak of 2θselected from 18.07° (±0.2°), 25.02° (±0.2°); more preferably, the X-ray powder diffraction pattern shows characteristic peaks of 2θ of 7.06° (±0.2°) and 20.87° (±0.2°), and at least one characteristic peak of 2θ selected from 18.07° (±0.2°), 17.58° ( ±0.2°), 10.54° (±0.2°), 23.91° (±0.2°), and 25.02° (±0.2°); more preferably, the X-ray powder diffraction pattern shows characteristic peaks of 2θ of 7.06° (±0.2°) and 20.87° (±0.2°), and at least one characteristicpeakof 2θ selected from 18.07° (±0.2°), 17.58° (±0.2°), 10.54° (±0.2°), 23.91° (±0.2°), 27.65° (±0.2°), 27.05° (±0.2°), 21.68° (±0.2°), 25.91° (±0.2°), and 25.02° (±0.2°); and, more preferably, the X-ray powder diffraction pattern shows the characteristic peaks of 2θ as shown in Table 1-1 below; the solid form of the sodium salt of the compound of formula (I) shows an X-ray powder diffraction pattern substantially identical to that of FIG. 10.

**Table 1-1**

| Angler | d Value | Rel. Intensity |
|---|---|---|
| 34.481 ° | 2.59903 Å | 1.9% |
| 39.178 ° | 2.9754 Å | 2.2% |
| 13.592 ° | 6.50929 Å | 2.9% |
| 31.870 ° | 2.80574 Å | 3.2% |
| 12.321 ° | 7.17776 Å | 3.4% |
| 34.678 ° | 2.58470 Å | 3.8% |
| 30.448 ° | 2.93348 Å | 4.1% |
| 28.287 ° | 3.15246 Å | 4.2% |
| 36.676 ° | 2.44834 Å | 4.4% |
| 12.555 ° | 7.04456 Å | 4.9% |
| 31.371 ° | 2.84918 Å | 4.9% |
| 35.305 ° | 12.54019 Å | 5.3% |
| 32.470 ° | 2.75521 Å | 6.2% |
| 18.807 ° | 4.71459 Å | 7.9% |
| 33.455 ° | 2.67631 Å | 7.9% |
| 29.893 ° | 2.98662 Å | 8.5% |
| 38.331 ° | 2.34635 Å | 8.8% |
| 25.384 ° | 3.50600 Å | 11.6% |
| 14.083 ° | 6.28381 Å | 12.2% |
| 16.229 ° | 5.45731 Å | 12.9% |
| 22.633 ° | 3.92552 Å | 15.7% |
| 7.066 ° | 12.49949 Å | 17.9% |
| 37.114 ° | 2.42042 Å | 18.9% |
| 25.911 ° | 3.43584 Å | 20.2% |
| 21.679 ° | 4.09613 Å | 21.0% |
| 27.049 ° | 3.20388 Å | 22.9% |
| 27.654 ° | 3.22319 Å | 23.2% |
| 23.910 ° | 3.71862 Å | 26.8% |
| 10.546 ° | 8.38159 Å | 31.8% |
| 17.584 ° | 5.03962 Å | 39.8% |
| 25.019 ° | 3.55635 Å | 49.1% |
| 18.073 ° | 4.90447 Å | 99.6% |
| 20.875 ° | 4.25200 Å | 100.0% |

As a solid form (crystal form D) of the sodium salt of the compound of formula (I), a differential scanning calorimetry curve thereof has an endothermic peak at 183.79°C (±3°C) with an enthalpy value of 3.9256 J/g and a starting temperature of 175.04°C, and showsan exothermic peak at 210.79°C (±3°C); more preferably, the solid form of the sodium salt of a compound of formula (I) shows a differential scanning calorimetry curve patternsubstantially identical to that of FIG. 11.

As a solid form of the sodium salt of the compound of formula (I) (crystal form D), the thermogravimetric analysis curve thereof shows that the sample has no weight loss prior to decomposition; more preferably, the solid form of the sodium salt of the compound of formula (I) shows a thermogravimetric analysis curve pattern substantially identical to that of FIG. 12.

### (2) Potassium salt of the compound of formula (I)

In the present invention, the solid form of the sodium salt of the compound of formula (I) refers to the salt obtained by contact reaction of the compound of formula (I) with the containing-potassiumbase. It preferably has a structure as shown in formula (III), with x selected from 0 .5-2, such as 0.5, 0.6, 0.7, 0.8, 0.9, 1.0, 1.1, 1.2, 1.3, 1.4, 1.5, 1.6, 1.7, 1.8, 1.9, or 2.0.

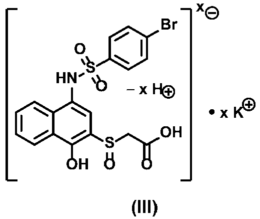

As a solid form of the potassium salt of the compound of formula (I), the X-ray powder diffraction pattern thereof shows characteristic peaks of 2θ of 9.67° (±0.2°) and 19.63° (±0.2°); more preferably, the X-ray powder diffraction pattern thereof shows characteristic peaks of 2θ of 9.67° (±0.2°) and 19.63° (±0.2°) , as well as at least one characteristicpeak of 2θselected from 11.34 ° (±0.2°), 16.68° (±0.2°) and 22.46° (±0.2°) ; more preferably, the solid form of the potassium salt of the compound of formula (I) shows an X-ray powder diffraction pattern substantially identical to that of FIG. 16.

As a solid form of the potassium salt of the compound of formula (I), the differential scanning calorimetry curve thereof shows a differential scanning calorimetry curve pattern substantially identical to that of FIG. 17.

As a solid form of the potassium salt of the compound of formula (I), the thermogravimetric analysis curve thereof shows a thermogravimetric analysis curve pattern substantially identical to that of FIG. 18.

### (3) Calcium salt of the compound of formula (I)

In the present invention, the solid form of the calcium salt of the compound of formula (I) refers to the salt obtained by contact reaction of the compound of formula (I) with the calcium-containing base. It preferably has a structure as shown in formula (IV), with x selected from 0 .5-2, such as 0.5, 0.6, 0.7, 0.8, 0.9, 1.0, 1.1, 1.2, 1.3, 1.4, 1.5, 1.6, 1.7, 1.8, 1.9, or 2.0.

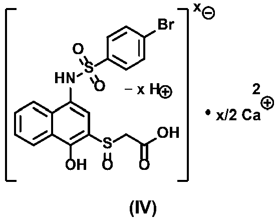

As a solid form of the calcium salt of the compound of formula (I), the X-ray powder diffraction pattern thereof shows characteristic peaks of 2θ of 11.53° (±0.2°) and 21.54° (±0.2°); more preferably, the X-ray powder diffraction pattern thereof shows characteristic peaks of 2θ of 11.53° (±0.2°) and 21.54° (±0.2°), and at least one characteristicpeakof 2θ selected from 12.46° (±0.2°), 18.56° (±0.2°) , 20.92° (±0.2°), 25.20° (±0.2°) and 26.06° (±0.2°) 2θ ; more preferably, the solid form of the calcium salts of the compounds of formula (I) shows an X-ray powder diffraction pattern substantially identical to that of Figure 19.

As a solid form of the calcium salt of the compound of formula (I), the differential scanning calorimetry curve thereof shows a differential scanning calorimetry curve pattern substantially identical to that of FIG. 20.

As a solid form of a calcium salt of a compound of formula (I), a thermogravimetric analysis curve thereof shows a thermogravimetric analysis curve pattern substantially identical to that of FIG. 21.

### (4) Tromethamine salt of the compound of formula (I)

In the present invention, the solid form of the tromethamine salt of the compound of formula (I) refers to the salt obtained by contact reaction of the compound of formula (I) with the tromethamine-containing base. It preferably has a structure as shown in formula (V), with x selected from 0 .5-2, such as 0.5, 0.6, 0.7, 0.8, 0.9, 1.0, 1.1, 1.2, 1.3, 1.4, 1.5, 1.6, 1.7, 1.8, 1.9, or 2.0.

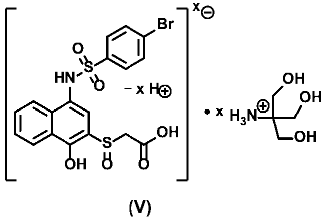

As a solid form of the tromethamine salt of the compound of formula (I), the X-ray powder diffraction pattern thereof shows characteristic peaks of 2θ of 8.94° (±0.2°) and 14 .35° (±0.2°); more preferably, the X-ray powder diffraction pattern thereof shows characteristic peaks of 2θ of 8.94° (±0.2°) and 14 .35° (±0.2°), and at least one characteristicpeak of 2θ selected from 17.09° (±0.2°), 18.69° (±0.2°), 19.76° (±0.2°), 22.19° (±0.2°), 22.59° (±0.2°), 22.99° (±0.2°), 25.40° (±0.2°), and 26.58° (±0.2°) ; more preferably, the solid form of the tromethamine salt of the compound of formula (I) shows an X-ray powder diffraction pattern substantially identical to that of FIG. 22.

As a solid form of the tromethamine salt of the compound of formula (I), the differential scanning calorimetry curve thereof shows a differential scanning calorimetry curve pattern substantially identical to that of FIG. 23

As a solid form of antromethamine salt of a compound of formula (I), the thermogravimetric analysis curve thereof shows a thermogravimetric analysis curve pattern substantially identical to that of FIG. 24.

### (5) Lysine salt of the compound of formula (I)

In the present invention, a solid form of the lysine salt of the compound of formula (I) refers to the salt obtained by contact reaction of the compound of formula (I) with the lysine-containing base. It preferably has a structure as shown in formula (VI) with x of 0.5-2.0. For example, x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1 or 1.1.

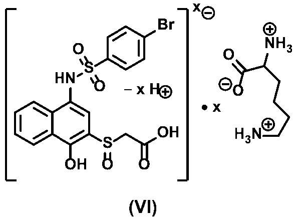

As a solid form of the lysine salt of the compound of formula (I), the X-ray powder diffraction pattern thereof shows characteristic peaks of 2θ of 12.31° (±0.2°) and 19.66° (±0.2°); more preferably, the X-ray powder diffraction pattern thereof shows characteristic peaks of 2θ of 12.31° (±0.2°) and 19.66° (±0.2°), as well as at least one characteristic peakof 2θ selected from the group of 17.72° (±0.2°), 21.16° (±0.2°), and 24.13° (±0.2°); more preferably, the solid form of the lysine salt of the compound of formula (I) shows an X-ray powder diffraction pattern substantially identical to that of FIG. 25.

As a solid form of the lysine salt of the compound of formula (I), the thermogravimetric analysis curve thereofshows a thermogravimetric analysis curve pattern substantially identical to that of FIG. 27.

### (6) Tert-butylamine salt of the compound of formula (I)

In the present invention, a solid form of a tert-butylamine salt of the compound of formula (I) refers to the salt obtained by contact reaction of the compound of formula (I) with the base containing tert-butylamine. It preferably has a structure as shown in formula (VII) with x of 0.5-2.0. For example, x is 0.5, 0.6, 0.7, 0.8, 0.9, 1.0, 1.1, 1.2, 1.3, 1.4, 1.5, 1.6, 1.7, 1.8, 1.9 or 2.0. Preferably, x is 0.8, 0.9, 1 or 1.1.

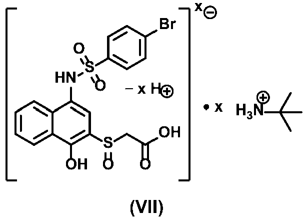

As a solid form of the tert-butylamine salt of the compound of formula (I), the X-ray powder diffraction pattern thereof shows characteristic peaks of 2θ of 7.15° (±0.2°) and 10.14° (±0.2°); more preferably, the X-ray powder diffraction pattern thereof shows characteristic peaks of 2θ of 7.15° (±0.2°) and 10.14° (±0.2°) , and at least one characteristic peak of 2θ selected from 17.85° (±0.2°), 18.28° (±0.2°), 19.19° (±0.2°), 20.51° (±0.2°), and 22.03° (±0.2°); more preferably, the solid form of the tert-butylamine salt of the compound of formula (I) shows an X-ray powder diffraction pattern substantially identical to FIG. 28.

As a solid form of the tert-butylamine salt of the compound of formula (I), the differential scanning calorimetry curve thereof shows a differential scanning calorimetry curve pattern substantially identical to that of FIG. 29.

As a solid form of a tert-butylamine salt of a compound of formula (I), a thermogravimetric analysis curve thereof shows a thermogravimetric analysis curve pattern substantially identical to that of FIG. 30.

### (7) Diisopropylamine salt of the compound of formula (I)

In the present invention, the solid form of the diisopropylamine salt of the compound of formula (I) refers to the salt obtained by contact reaction of a compound of formula (I) with the base containing diisopropylamine. It preferably has a structure as shown in formula VIII) with x of 0.5-2.0. For example, x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

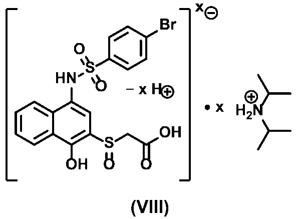

As a solid form of the diisopropylamine salt of the compound of formula (I), the X-ray powder diffraction pattern thereof shows characteristic peaks of 2θ of 8.87° (±0.2°) and 17.20° (±0.2°); more preferably, the X-ray powder diffraction pattern thereof shows characteristic peaks of 2θ of 8.87° (±0.2°) and 17.20° (±0.2°), and at least one characteristic peak of 2θ selected from9.25° (±0.2°), 9.65° (±0.2°), 15.07° (±0.2°), 16.97° (±0.2°), 18.06° (±0.2°), 19.31° (±0.2°), 20.01° (±0.2°), 22.65° (±0.2°), and 27.29° (±0.2°); more preferably, the solid form of the diisopropylamine salt of the compound of formula (I) shows an X-ray powder diffraction pattern substantially identical to that of FIG. 31.

As a solid form of the diisopropylamine salt of the compound of formula (I), the differential scanning calorimetry curve thereofshows a differential scanning calorimetry curvepattern substantially identical to that of FIG. 32.

As a solid form of a diisopropylamine salt of a compound of formula (I), a thermogravimetric analysis curve thereof shows a thermogravimetric analysis curve pattern substantially identical to that of FIG. 33.

### (8) Ethanolamine salt of the compound of formula (I)

In the present invention, the solid form of the ethanolamine salt of the compound of formula (I) refers to the salt obtained by contact reaction of the compound of formula (I) with the base containing ethanolamine. It preferably has a structure as shown in formula (IX) with x of 0.5-2.0. for example, x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

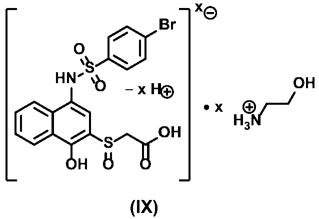

As a solid form of the ethanolamine salt of the compound of formula (I), the X-ray powder diffraction pattern thereof shows characteristic peaks of 2θ of 7.62° (±0.2°) and 19.70° (±0.2°); more preferably, the X-ray powder diffraction pattern thereof shows characteristic peaks of 2θ of 7.62° (±0.2°) and 19.70° (±0.2°) and at least one characteristic peak of 2θ selected from 9.72° (±0.2°), 14.98° (±0.2°), and 19.34° (±0.2°); more preferably, the solid form of the ethanolamine salt of the compound of formula (I) shows an X-ray powder diffraction pattern substantially identical to that of FIG. 34.

As a solid form of the ethanolamine salt of the compound of formula (I), the differential scanning calorimetry curve thereofshows a differential scanning calorimetry curvepattern substantially identical to that of FIG. 35.

As a solid form of an ethanolamine salt of a compound of formula (I), a thermogravimetric analysis curve thereof shows a thermogravimetric analysis curve pattern substantially identical to that of FIG. 36.

### (9) Diethanolamine salt of the compound of formula (I)

In the present invention, the solid form of the diethanolamine salt of the compound of formula (I) refers to the salt obtained by contact reaction of a compound of formula (I) with the base containing diethanolamine. It preferably has a structure as shown in formula (X), with x being 0.5-2.0. For example, x is 0.5, 0.6, 0.7, 0.8, 0.9, 1.0, 1.1, 1.2, 1.3, 1.4, 1.5, 1.6, 1.7, 1.8, 1.9, or 2.0. Preferably, x is 0.8, 0.9, 1, or 1.1.

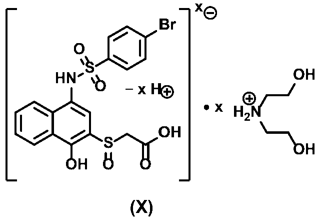

As a solid form of the diethanolamine salt of the compound of formula (I), the X-ray powder diffraction pattern thereof shows characteristic peaks of 2θ of 6.33° (±0.2°) and 19.87° (±0.2°); more preferably, the X-ray powder diffraction pattern thereof shows characteristic peaks of 2θ of 6.33° (±0.2°) and 19.87° (±0.2°), and at least one characteristic peak of 2θ selected from the group of 7.41° (±0.2°), 12.46° (±0.2°), 22.02° (±0.2°), and 28.45° (±0.2°); more preferably, the solid form of the diethanolamine salt of the compound of formula (I) shows an X-ray powder diffraction pattern substantially identical to that of FIG. 37.

As a solid form of the diethanolamine salt of the compound of formula (I), the differential scanning calorimetry curve thereofshows a differential scanning calorimetry curvepattern substantially identical to that of FIG. 38.

As a solid form of a diethanolamine salt of a compound of formula (I), a thermogravimetric analysis curve thereof shows a thermogravimetric analysis curve patternsubstantially identical to that of FIG. 39.

### (10) Solid form of the free acid of the compound of formula (I)

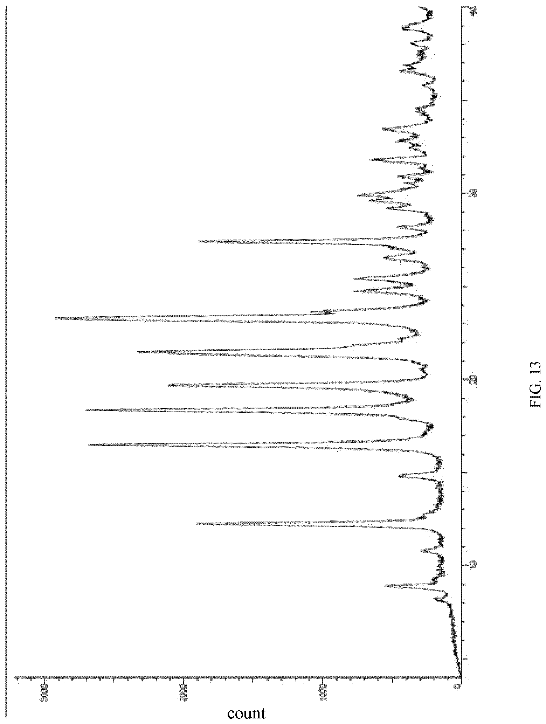

In the present invention, the solid form of the free acid of the compound of formula (I) refers to the solid of the compound of formula (I). As a solid form of the free acid of the compound of formula (I), the X-ray powder diffraction pattern thereof shows at least one characteristic peak of 2θ selected from 8.92° (±0.2°) and 23.31° (±0.2°); more preferably, the X-ray powder diffraction pattern of the solid form of the free acid of the compound of formula (I) shows at least one characteristic peak of 2θ selected from 8.92° (±0.2°), 23.31° (±0.2°), 27.41° (±0.2°), 19.70° (±0.2°), 16.51° (±0.2°), 12.27° (±0.2°), 21.50° (±0.2°), 18.23° (±0.2°), and 18.37° (±0.2°); more preferably, the solid form of the free acid of the compound of formula (I) shows an X-ray powder diffraction pattern substantially identical to that of FIG. 13.

As a solid form of the free acid of the compound of formula (I), the differential scanning calorimetry curve thereof shows a differential scanning calorimetry curve pattern substantially identical to that of FIG. 14.

As a solid form of the free acid of the compound of formula (I), a thermogravimetric analysis curve thereof shows a thermogravimetric analysis curve pattern substantially identical to that of FIG. 15.

### Pharmaceutical composition

The present invention also relates to a pharmaceutical composition comprising the solid form of the compound of formula (I), and a pharmaceutically acceptable carrier or excipient.

As used herein, the term "composition" means a pharmaceutical preparation suitable for administration to anparticular individual for therapeutic purposes, comprising at least one pharmaceutically active compound, including any solid or amorphous form thereof. The composition may include at least one pharmaceutically acceptable component to provide a modified formulation of the compound, such as a suitable carrier or excipient.

The term "pharmaceutically acceptable" indicates that the indicated material does not have properties that would cause a reasonably prudent medical practitioner to avoid administering the material to a patient, taking into account the disease or condition to be treated and the respective route of administration. For example, it is often desirable for such materials to be essentially sterile, e.g., for use in injectable agents.

### Uses

The present invention also relates to uses of the solid formofthe compound of formula (I) or pharmaceutical composition for (i) preparing a drug for preventing and/or treating diseases associated with kidney injury; and/or
(ii) preventing and/or treating diseases associated with kidney injury; and/or
(iii) preventing and/or treating diseases associated with mitochondrial dysfunction; and/or
(iv) preparing a drug for preventing and/or treating diseases associated with mitochondrial dysfunction.

In some preferred embodiments, the diseases associated with kidney injury comprises acute kidney injury and chronic kidney injury, preferably chronic kidney injury.

In some preferred embodiments, the diseases associated with kidney injury are selected from acute renal ischemia-reperfusion injury, septic nephropathy, nephrotoxic injury, primary glomerulonephritis, hypertensive renal microarteriosclerosis, diabetic nephropathy, secondary glomerulonephritis, tubulointerstitial lesions, ischemic nephropathy, and hereditary nephropathy.

In some preferred embodiments, the tubulointerstitial lesion is selected from chronic pyelonephritis, chronic uric acid nephropathy, obstructive nephropathy, and pharmacologic nephropathy.

In some preferred embodiments, the hereditary nephropathy is selected from polycystic kidney and hereditary nephritis.

In some preferred embodiments, the disease associated with mitochondrial dysfunction is selected from at least one of the following: inflammatory bowel disease; lung injury; fibrotic disease; sepsis; prostate disease; cardiovascular disease; neurological disease; and diseases associated with aging.

In some preferred embodiments, the inflammatory bowel disease is selected from at least one of ulcerative colitis and Crohn's disease.

In some preferred embodiments, the fibrotic disease is selected from at least one of renal tubulointerstitial fibrosis, interstitial lung disease (ILD), idiopathic pulmonary fibrosis (IPF), chronic obstructive pulmonary disease fibrosis, tissue fibrosis, arthrofibrosis, hepatic fibrosis, dermal fibrosis, fibromatosis, myelofibrosis, cardiac fibrosis, and cystic fibrosis disease.

In some preferred embodiments, the cardiovascular disease is selected from at least one of atherosclerosis, heart failure, myocardial ischemia/reperfusion injury, and hypertension, cardiomyopathy, and cardiovascular complications of diabetes.

In some preferred embodiments, the neurological disease is selected from at least one of sensorineural hearing loss, cerebral developmental anomalies, congenital hydrocephalus, congenital cranial nerve disorders, congenital brain-through-pathway malformations, metabolic dysfunctions, congenital auditory aphasia, congenital visual aphasia, cerebral palsy, mental disorders of depression, schizophrenia, bipolar disorder, paranoid disorder, mania, obsessive-compulsive disorder, autism and other mental illnesses, Parkinson's disease, Alzheimer's disease, brain injury, muscular dystrophy lateral sclerosis, epilepsy, Huntington's disease, spinal cerebellar ataxia, and cerebral ischemia.

In some preferred embodiments, the disease associated with aging is progeria.

In some preferred embodiments, the aging-related disease is skin aging, and more preferably, the aging-related disease is radiation-induced skin aging or damage.

In order to make the objects, technical solutions and advantages of the embodiments of the present invention clearer, the invention is further elaborated below in connection with specific embodiments. It should be understood that these embodiments are used only to illustrate the invention and are not intended to limit the scope of the invention. Experimental methods for which specific conditions are not indicated in the following embodiments are generally in accordance with conventional conditions, or in accordance with conditions recommended by the manufacturer. Unless otherwise indicated, percentages and parts are weight percentages and weight parts. Experimental materials and reagents used in the following embodiments are commercially available if not otherwise indicated.

Unless otherwise indicated, technical and scientific terms used herein have the same meanings as are commonly understood by one of ordinary skill in the art to which this application belongs, and it is to be noted that the terminology used herein is intended only to describe specific embodiments and is not intended to limit the exemplary embodiments of the present application.

### Embodiment 1

In this embodiment, a crystal form A of compound TJ01-013 (sodium salt of compound TJ) is prepared and obtained. The specific steps are as follows:
Weighing 0.300 g of compound of formula (I) into a 20 mL vial and adding 12 mL of acetone, heating to about 45°C, the sample is dissolved and clarified, adding 0.520 g of 10% aqueous sodium bicarbonate solution dropwise without stirring, the solid is precipitated out and sinks to the bottom, keeping warm for 30 minutes, slowly cooling to normal atmospheric temperature (about 20°C), leaching, and washing with a small amount of acetone, and drying in a vacuum at normal atmospheric temperature to obtain the sample of crystal form A.

### Embodiment 2

In this embodiment, a crystal form B of the compound TJ01-013 is prepared and obtained. The specific steps are as follows:
Weighing 0.300 g of compound of formula (I) into a 20 mL vial and adding 12 mL of acetone, heating to about 45°C, the sample is dissolved and clarified, cooling to normal atmospheric temperature (about 20°C), adding 0.520 g of 10% aqueous sodium bicarbonate dropwise, stirring magnetically (at a speed of 1,000 rpm), and after the solid is precipitated,keeping warm for 30 minutes, leaching, and washing with a small amount of acetone, to obtain the sample of crystal form B.

### Embodiment 3

In this embodiment, a crystal form C of the compound TJ01-013 is prepared and obtained. The specific steps are as follows:
Weighing 0.100 g of compound of formula (I) into a 20 mL vial and adding 4 mL of acetone, heating to about 45°C, the sample is dissolved and clarified, adding 0.173 g of 10% aqueous sodium bicarbonate solution dropwise, stirringmagnetically, after the solid is precipitated, stirring quickly and keeping warm for 30 minutes, cooling to normal atmospheric temperature (about 20°C), leaching, and washing with a small amount of acetone, and drying in a vacuum at ambient temperature to obtain the sample of crystal form C.

### Embodiment 4

In this Example, a crystal form D of the compound TJ01-013 is prepared and obtained by the following two methods.

### (1) Method One

Weighing 0.100 g of the compound of formula (I), adding 4 ml of acetone. Heating to about 45°C, stirring, dissolving and clarifying.Adding 0.223g of sodium methoxide solution in methanol dropwise at about 45°C,a small amount of solid exists when adding dropwise, dissolving and clarifyingafter stirring. After stirring, the solid is slowly precipitated, keeping warm for 1hat about 45 °C. Whenkeeping warm completes, cooling to about 20 °C, stirring for 1 h. Leaching, and washing with a small amount of acetone, vacuum drying at normal atmospheric temperature (without heating) for about 20h. Detecting the output material as crystal form D.

**Table 2-1 Ratio of materials**

| Material name | Amount of material | Molecularweight | Source |
|---|---|---|---|
| Compound of formula (I) | 0.100 | 484.34 | E020388-072-12 |
| 5% sodium methoxide methanol solution | 0.223 | 54 | Self-prepared |
| Acetone | 4ml | N/A | 5055R210601M |

### (2) Method Two

Weighing 3.00 g of compound of formula (I) and adding 120 ml of acetone, heating it to about 45°C, stirring, dissolving and clarifying. Adding 5.20g of 10% aqueous sodium bicarbonate solution dropwise at about 45°C,a small amount of solid exists when adding dropwise, dissolving and clarifyingafterstirring.Afterstirring,thesolid is slowly precipitated, keeping warm for 1hat about 45 °C. Whenkeeping warm completes, cooling to about 20 °C, stirring for 1 h. Leaching, and washing with a small amount of acetone. Vacuum drying at normal atmospheric temperature (without heating) for about 20h. Detecting the output material as crystal form D.

**Table 2-2 Ratio of materials**

| Material name | Amount of material | Molecularweight | Source |
|---|---|---|---|
| Compound of formula (I) | 3.00 | 484.34 | E020388-103-15 |
| 10% aqueous sodium bicarbonate solution | 5.20 | 84 | Self-prepared |
| Acetone | 120ml | N/A | 5055R220201M |

### Embodiment5

In this embodiment, a potassium salt crystal form F of the compound TJ01-013 is prepared and obtained. The specific steps are as follows:
Weighing 0.3 g of compound of formula (I), adding 12 mL of acetone, warming to about 45°C, dissolving and clarifying it, adding 0.62 g of 10% potassium bicarbonate, after dissolving and clarifying it, a solid (a small amount of crystalline seed can be added) is precipitated, cooling to about 20°C, and leaching under suction.

### Embodiment6

In this embodiment, a calcium salt crystal form G of the compound TJ01-013 is prepared and obtained. The specific steps are as follows:
Weighing 0.3 g of compound of formula (I), adding 12 mL of acetone, heatingto about 45°C to dissolve and clarify it, adding 10% calcium acetate 0.98 g, after dissolving and clearing it, a crystal is not precipitated, blowing dry the solvent with nitrogen, adding 12 mL of isopropanol, and a solid is precipitated, cooling to about 20 °C, and leaching under suction.

### Embodiment 7

In this embodiment, a tromethamine salt crystal form H of the compound TJ01-013 is prepared and obtained. The specific steps are as follows:
Weighing 0.3 g of compound of formula (I), adding 10 mL of methanol, heatingto about 45°C to dissolve and clarify it, adding 1.5 g of 5% aqueous tromethamine solution and keeping warm for 0.5~1h, blowingdry the solvent with nitrogen, adding 10 mL of ethyl acetate, and a solid is precipitated, cooling to about 20°C, and leaching under suction.

### Embodiment 8

In this embodiment, a lysine salt crystal form I of the compound TJ01-013 is prepared and obtained. The specific steps are as follows:
Weighing 0.05 g of compound of formula (I), adding 3 mL of acetone, heatingto about 45°C to dissolve and clarify it, adding 0.30 g of 5% aqueous lysine solution, after dissolving and clarifying, cooling to precipitate a solid, then cooling it to about 20°C, and leaching under suction.

### Embodiment 9

In this embodiment, a tert-butylamine salt crystal form J of the compound TJ01-013 is prepared and obtained. The specific steps are as follows:
Weighing 0.3 g of compound of formula (I), adding 12 mL of acetone, heatingto about 45°C to dissolve and clarify, adding 5% tert-butylamine acetone solution 0.906 g, after dissolving and clarifying, cooling to precipitate a solid, then cooling to about 20°C, and leaching under suction.

### Embodiment 10

In this embodiment, a diisopropylamine saitcrystal form K of the compound TJ01-013 is prepared and obtained. The specific steps are as follows:
Weighing 0.3 g of compound of formula (I), adding 12 mL of acetone, heatingto about 45°C to dissolve and clarify it, adding 5% diisopropylamine acetone solution 1.253 g, after dissolving and clarifying, cooling to precipitate a solid, then cooling to about 20°C, and leaching under suction.

### Embodiment 11

In this embodiment, an ethanolamine slat crystal form L of the compound TJ01-013 is prepared and obtained. The specific steps are as follows:
Weighing 0.3 g of compound of formula (I), adding 12 mL of acetone, heatingto about 45°C to dissolve and clarify it, adding 0.756 g of 5% ethanolamine acetone solution, after dissolving and clarifying it, cooling to precipitate a solid, then cooling to about 20°C, and leaching under suction.

### Embodiment 12

In this embodiment, a diethanolamine slat crystal form M of the compound TJ01-013 is prepared and obtained. The specific steps are as follows:
Weighing 0.3 g of compound of formula (I), adding 12 mL of acetone, heating to about 45°C to dissolve and clarify it, adding 5% diethanolamine acetone solution 1.302 g, after dissolving and clarifying it, cooling to precipitate a solid, then cooling to about 20°C, and leaching under suction.

### Embodiment 13

In this embodiment, a free acid crystal form E the compound TJ01-013 is prepared and obtained. The specific steps are as follows:

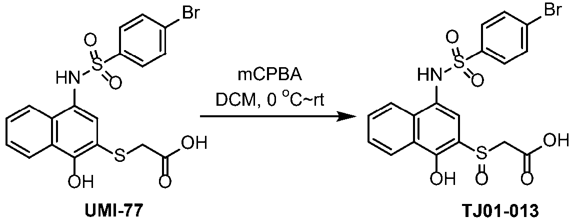

Suspending UMI-77 (15 mg, 0.032 mmol) in DCM (0.5 mL) and placing in an ice water bath with stirring, adding 85% mCPBA (6.5 mg, 0.032 mmol), followed by a slow return to room temperature. Leaching under suction, and washing the filter cake with DCM, and drying to obtain the product TJ01-013 (crystal form E, 6 mg).

X-ray diffraction, differential scanning calorimetry and thermogravimetric analysis are used in the present invention to characterize the samples of the crystal forms obtained and prepared in the above embodiments.

### [X-ray diffraction method]

Thecrystal forms prepared above are analyzed using a PANalytacal Empyrean X-ray powder diffraction analyzer. The scanning parameters are shown in Table 3 below.

**Table 3**

| Parameters | XRPD(reflection mode) |
|---|---|
| X-ray | Cu, kα, Kα1 (Å): λ =1.540598; Kα2 (Å): λ =1.544426 |
| | Kα2/Kα1 intensity ratio: 0.50 |
| Setting of X-ray tube | 30 kV,10 mA |
| Emission slit | 1.0mm |
| Acceptance slit | 3mm |
| Scan mode | Continuous |
| Scanning range(°2Theta) | 4°~40° (2θ) |
| Scanning step(°2Theta) | 0.02° (2θ) |
| Scanning rate(s/step) | 0.2 s/step |

The peak positions and intensities of the characteristic peaks of the X-ray powder diffraction pattern of the A crystal form are shown in Table 4-1 below, and the XRPD pattern is shown in Fig. 1.

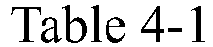

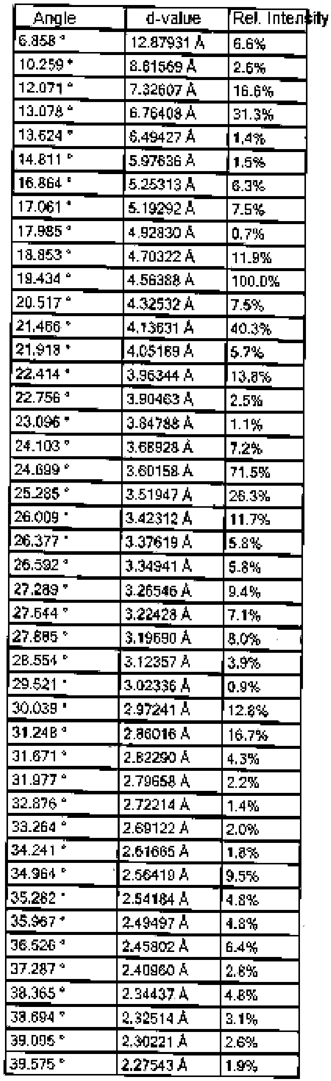

The peak positions and intensities of the characteristic peaks of the X-ray powder diffraction pattern of the B crystal form are shown in Table 4-2 below, and the XRPD pattern is shown in Fig.4.

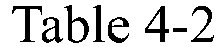

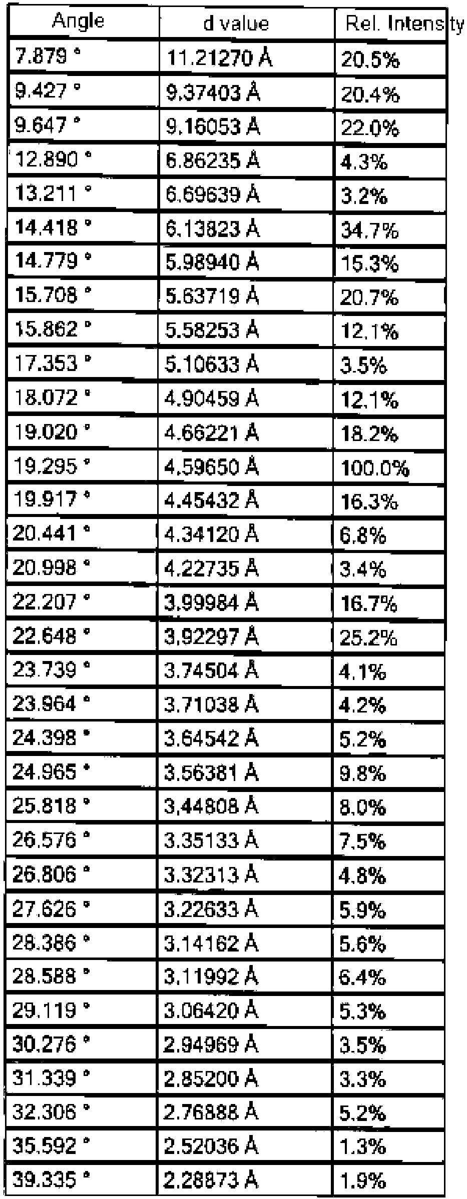

The peak positions and intensities of the characteristic peaks of the X-ray powder diffraction pattern of the C crystal form are shown in Table 4-3 below, and the XRPD pattern is shown in Fig.7.

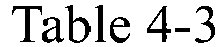

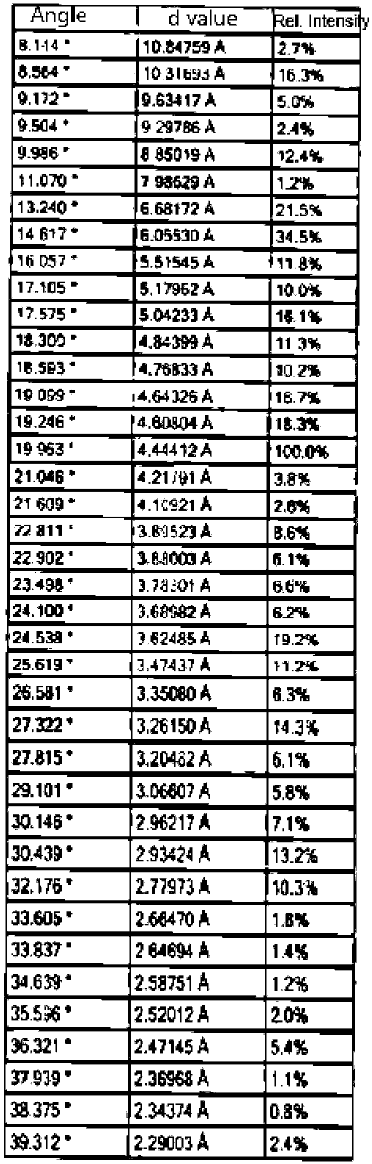

The peak positions and intensities of the characteristic peaks of the X-ray powder diffraction pattern of the D crystal form are shown in Table 4-4 below, and the XRPD pattern is shown in Fig.10.

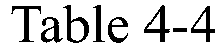

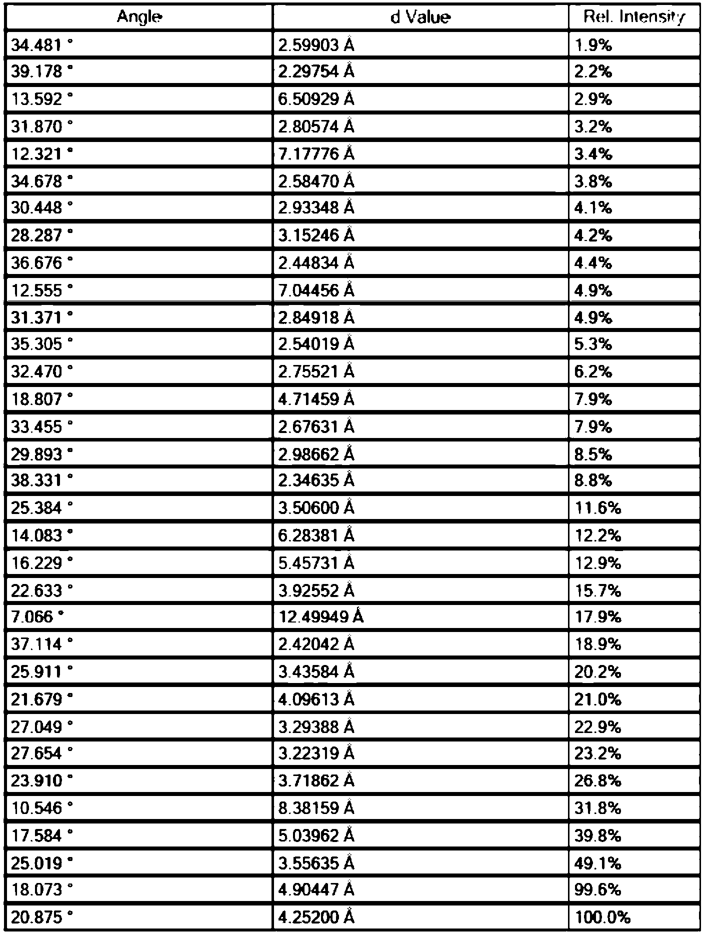

The peak positions and intensities of the characteristic peaks of the X-ray powder diffraction pattern of the E crystal form are shown in Table 4-5 below, and the XRPD pattern is shown in Fig.13.

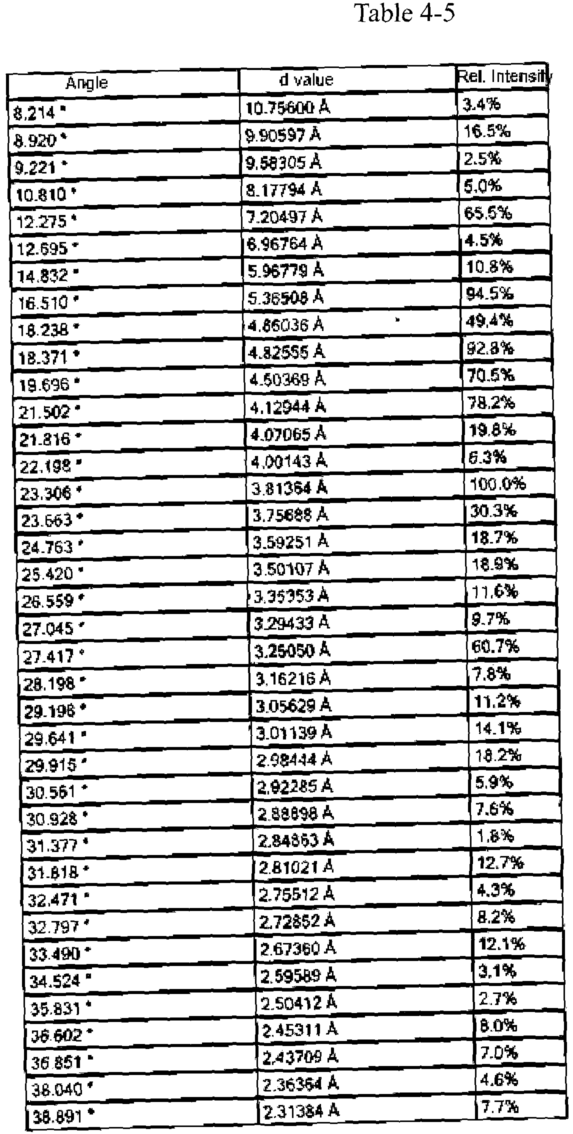

The peak positions and intensities of the characteristic peaks of the X-ray powder diffraction pattern of the F crystal form are shown in Table 4-6 below, and the XRPD pattern is shown in Fig.16.

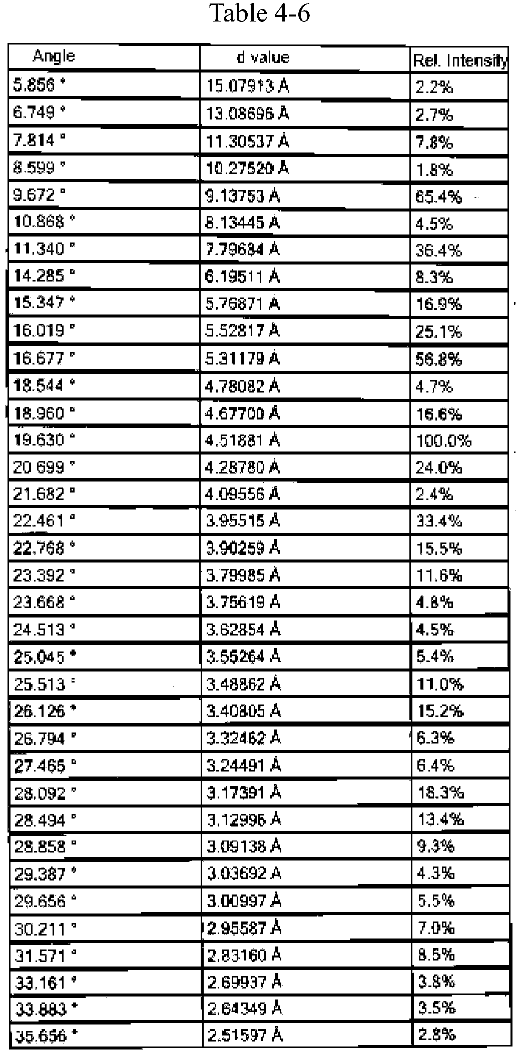

The peak positions and intensities of the characteristic peaks of the X-ray powder diffraction pattern of the G crystal form are shown in Table 4-7 below, and the XRPD pattern is shown in Fig.19.

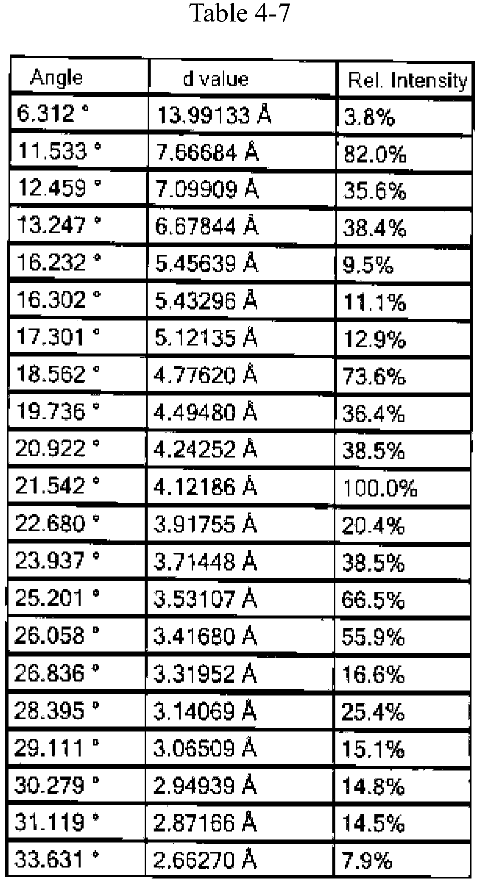

The peak positions and intensities of the characteristic peaks of the X-ray powder diffraction pattern of the H crystal form are shown in Table 4-8 below, and the XRPD pattern is shown in Fig.22.

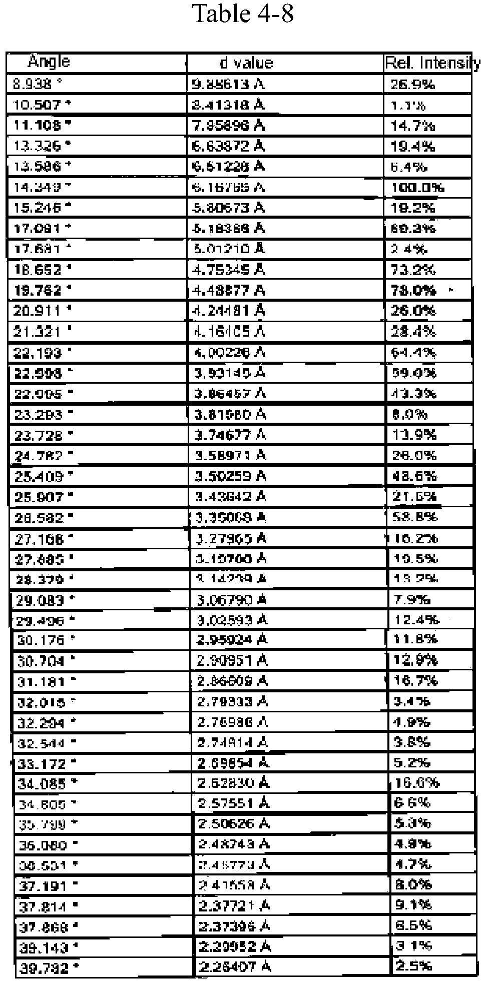

The peak positions and intensities of the characteristic peaks of the X-ray powder diffraction pattern of the I crystal form are shown in Table 4-9 below, and the XRPD pattern is shown in Fig.25.

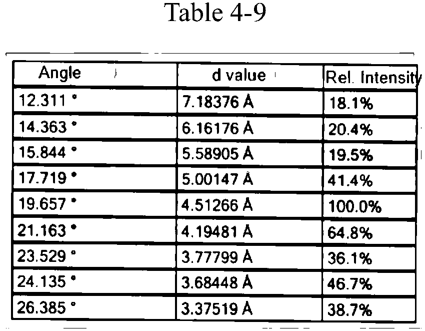

The peak positions and intensities of the characteristic peaks of the X-ray powder diffraction pattern of the J crystal form are shown in Table 4-10 below, and the XRPD pattern is shown in Fig.28.

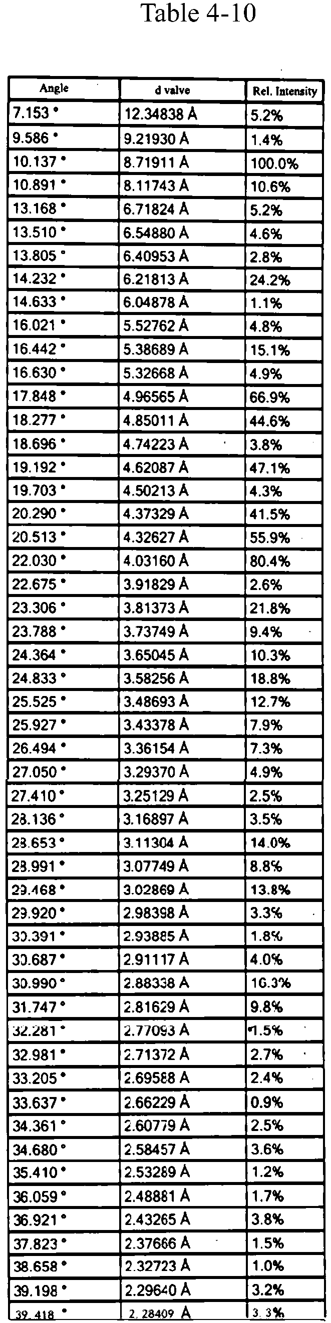

The peak positions and intensities of the characteristic peaks of the X-ray powder diffraction pattern of the K crystal form are shown in Table 4-11 below, and the XRPD pattern is shown in Fig.31.

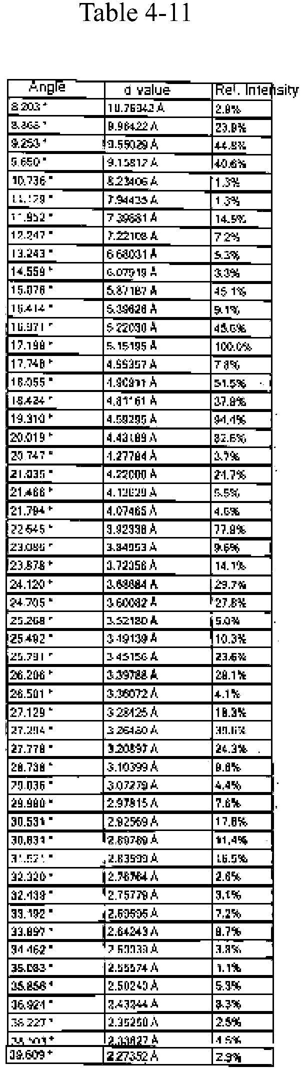

The peak positions and intensities of the characteristic peaks of the X-ray powder diffraction pattern of the L crystal form are shown in Table 4-12 below, and the XRPD pattern is shown in Fig.34.

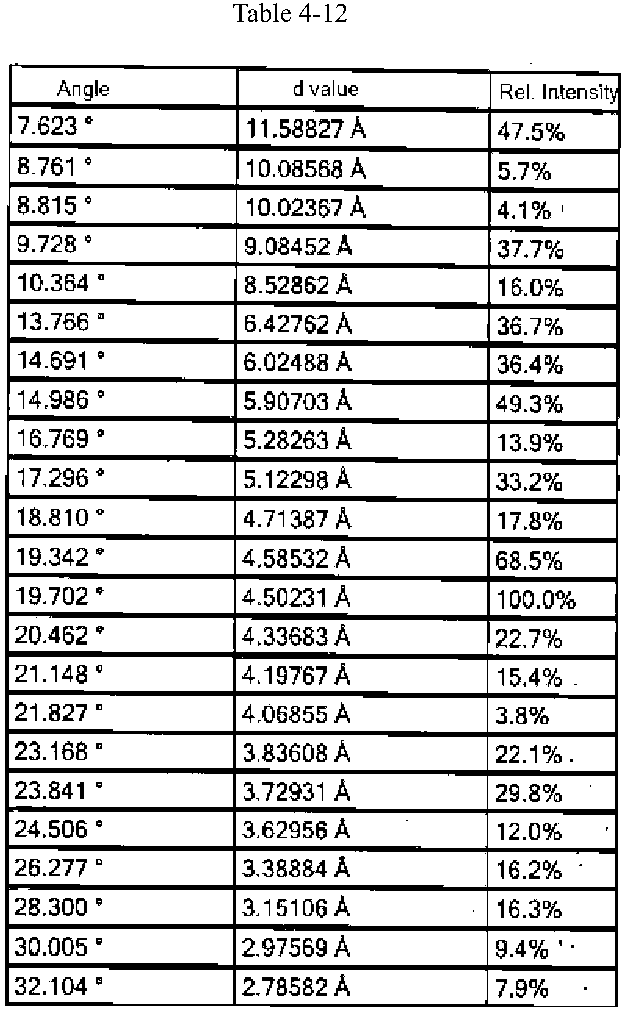

The peak positions and intensities of the characteristic peaks of the X-ray powder diffraction pattern of the M crystal form are shown in Table 4-13 below, and the XRPD pattern is shown in Fig.37.

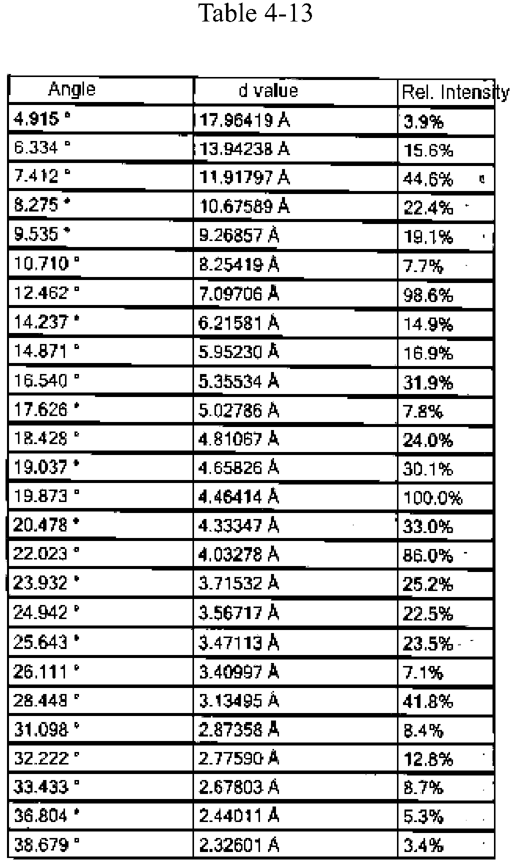

### [Differential Scanning Calorimetry]

The crystals prepared in the above embodiments are tested using a TA Q200/2000 differential scanning calorimeter, and the test parameters are shown in Table 5 below.

**Table 5**

| Parameters | | DSC |
|---|---|---|
| Method | Linear temperature increase | |
| Sample dish | Aluminum crucible, covered, cover with punctures | |
| Temperature range | | 30°C-250°C |
| Scanning rate (°C/min) | | 10 |
| Protective gas | | Nitrogen |

The DSC pattern of the A crystal form is shown in Fig. 2, and the differential scanning calorimetry curve of the A crystal form has an endothermic peak at 144.89°C±3°C and 150.40°C±3°C and an exothermic peak at 214.79°C±3°C.

The DSC pattern of B crystal form is shown in Fig. 5, the differential scanning calorimetry curve of B crystal form has an endothermic peak at 72.14°C ± 3°C and 145.82°C ± 3°C and an exothermic peak at 184.61°C ± 3°C.

The DSC pattern of C crystal form is shown in Fig. 8, and the differential scanning calorimetry curve of C crystal form has an endothermic peak at 59.75°C ± 3°C and 140.91°C ± 3°C and an exothermic peak at 185.61°C ± 3°C.

The DSC pattern of the D crystal form is shown in Fig. 11, and the differential scanning calorimetry curve of the D crystal form has an endothermic peak at 183.79°C (±3°C) and an exothermic peak at 210.79°C ±3°C.

The DSC pattern of E crystal form is shown in Fig. 14, and the differential scanning calorimetry curve of the E crystal form has an endothermic peak at 184.18 ± 3 °C and an exothermic peak at 198.31 °C ± 3 °C.

The DSC pattern of F crystal form is shown in Fig. 17.

The DSC pattern of G crystal formis shown in Fig. 20.

The DSC pattern of H crystal formis shown in Fig. 23.

The DSC pattern of J crystal formis shown in Fig. 29.

The DSC pattern of K crystal formis shown in Fig. 32.

The DSC pattern of L crystal formis shown in Fig. 35.

The DSC pattern of M crystal formis shown in Fig. 38.

### [thermogravimetric analysis]

The crystals prepared in the above embodiments are tested using a TA Q500/5000 thermogravimetric analyzer, and the test parameters are shown in Table 6 below.

**Table 6**

| Parameters | | TGA |
|---|---|---|
| Method | Linear temperature increase | |
| Sample dish | Aluminum crucible, covered, cover with punctures | |
| Temperature range | | 30°C-250°C |
| Scanning rate (°C/min) | | 10 |
| Protective gas | | Nitrogen |

The TGA pattern of the B crystal form is shown in Fig.6, and the thermogravimetric analysis curve of the B crystal form has a weight loss of 6.78±0.2% at 73.50±3°C.

The TGA pattern of C crystal form is shown in Fig.9, and the thermogravimetric analysis curve of C crystal form has a weight loss of 4.96 ± 0.2% at 70.15 ± 3 °C.

The TGA pattern of the D crystal form is shown in Fig.12, and the thermogravimetric analysis curve of the D crystal form shows no weight loss of this sample before decomposition.

The TGA pattern of E crystal form is shown in Fig. 15.

The TGA pattern of crystal form F is shown in Fig. 18.

The TGA pattern of crystal form G is shown in Fig. 21.

The TGA pattern of crystal form H is shown in Fig. 24.

The TGA pattern of crystal form I is shown in Fig. 27.

The TGA pattern of crystal form J is shown in Fig. 30.

The TGA pattern of crystal form K is shown in Fig. 33.

The TGA pattern of crystal form L is shown in Fig. 36.

The TGA pattern of the crystal form M is shown in FIG. 39.

In addition, the properties of the samples of the crystal forms obtained and prepared in the above embodiments are studied in the present invention.

### [hygroscopicity]

Single crystalline sample of compound TJ01-013 is tested using a Waters vaporsorptionanalyser. Taking a dry weighingtray, and laying a single sample of crystal form flat in the weighing tray, and the volume of the loaded samples is about 1/3 to 2/3 of that of the weighing tray. Instrument parameters are set as follows: temperature: 25 °C; equilibrium: dm/dt = 0.01% / min (minimumtime: 10 minutes, and maximumtime: 180 minutes); drying: drying 120 minutes under 0% RH; RH (%) testinggradient: 10%; RH (%) test gradient range: 0% -90% - 0%. The hygroscopicity of each crystalline sample is tested at 90% RH, and the results are shown in Table 7 below.

**Table 7**

| Group | Crystal Form | Percentage weight gain by moisture absorption |
|---|---|---|
| Embodim ent 4 | sodium salt, Compound TJ01-013, D crystal form | 2.13% |
| Embodim ent 5 | potassium salt, Compound TJ01-013, F crystal form | 2.655% |
| Embodim ent 6 | calcium salt, Compound TJ01-013, G crystal form | 7.275% |
| Embodim ent 9 | tert-butylamine salt, Compound TJ01-013, J crystal form | 0.7884% |
| Embodim ent 10 | diisopropylaminesalt, Compound TJ01-013, K crystal form | 0.2033% |
| Embodim ent 11 | ethanolamine Salt, Compound TJ01-013, L crystal form | 1.897% |
| Embodim ent 12 | diethanolamine Salt, Compound TJ01-013, M crystal form | 6.026% |
| Embodim ent 7 | aminotriol salt, compound TJ01-013, H crystal form | 2.313% |
| Embodim ent 13 | E crystal form of the compound of Formula (I) | 0.439% |

According to Table 7, the sodium salt D crystal form of compound TJ01-013, the potassium salt F crystal form of compound TJ01-013, the tert-butylamine salt J crystal form of compound TJ01-013, the diisopropylamine salt K crystal form of compound TJ01-013, the ethanolamine salt L crystal form of compound TJ01-013, the tromethamine salt H crystal form of compound TJ01-013, E crystal form of the compound of Formula (I) are slightly hygroscopic, and the remaining crystal forms are more hygroscopic.

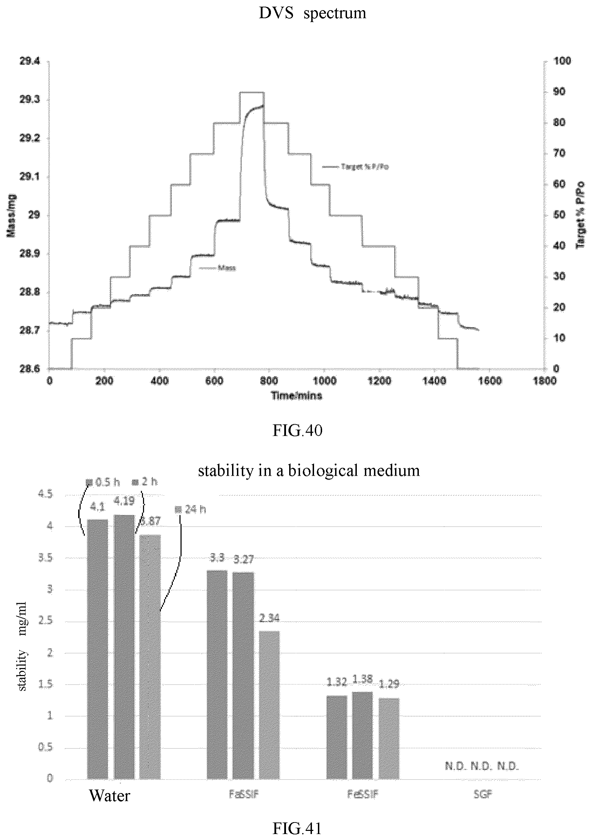

FIG. 40 exemplarily shows the DVS pattern of a sample of the sodium salt D crystal form of compound TJ01-013.

### [Solubility]

Aliquots of the samples are placed in the media FaSSIF buffer, FeSSIF buffer, water and SGF and tested for solubility at 0.5, 2 and 24 hours, respectively. The results of samples of D crystal form of compound TJ01-013 are shown in Table 8.

**Table 8**

| | | | |
|---|---|---|---|
| Medium | 0.5h | 2h | 24h |
| | Solubility(mg/mL) | Solubility(mg/mL) | Solubility(mg/mL) |
| FaSSIF | 3.30 | 3.27 | 2.34 |
| FeSSIF | 1.32 | 1.38 | 1.29 |
| Water | 4.10 | 4.19 | 3.87 |
| SGF | Not detected | Not detected | Not detected |

According to Fig.41, the D crystal form of compound TJ01-013 has high solubility in water, FaSSIF, FeSSIF and low solubility in SGF.

### [Crystal formconversion]

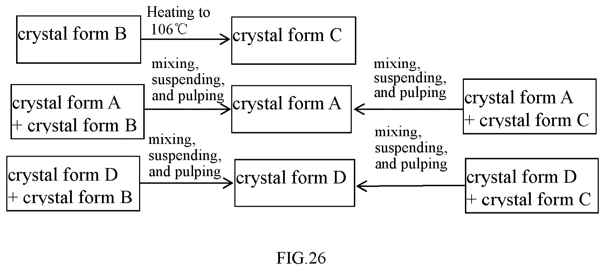

The mutual conversion relationship among the A, B, C, D crystal formsof the sodium salt is further studied in this embodiment by heat treatment under nitrogen protection and suspension competition test. The experimental results show that crystal form B can be converted into crystal form C by heating to 106°C and then cooling to room temperature. The competition test after physical mixing and stirring of equal amounts of four crystalline samples at different temperatures (RT/50) shows that after magnetic stirring (~1000 rpm) for about 20 hours, crystal form A and crystal form B are able to be converted into crystal form A by mixing and beating, crystal form A and crystal form C are able to be converted into crystal form Aby mixing and beating,, and crystal form B and crystal form D are able to be converted into crystal form D by mixing and beating,andcrystal form C and crystal form D are able to be converted into crystal form Dby mixing. That is, crystal forms B, C are able to be converted into crystal form A, D, which means that crystal form A, D are more stable than crystal form B, C. The conversion relationship of each crystal form is shown in Fig.26.

However, the methods of heating, suspension pulpingas well as grinding can not study the thermodynamic conversion relationship betweencrystal form A and crystal form D. In order to further study the thermodynamic conversion relationship of crystal form A and crystal form D, analyzed from the DSC pattern, the DSC of crystal form A is shown in Fig. 2, and the DSC of crystal form D is shown in Fig. 11, the melting point of crystal form A is higher than that of crystal form D, whereas the enthalpy of melting of crystal form A is much lower than that of crystal form D. It may be judged from the thermodynamic stability relationship table that if TA > TD, ΔHf, A >ΔHf, D, crystal form A and D is a univariate relationship, crystal form A is stable, if TA > TD, ΔHf, A <ΔHf, D, crystal forms A and D are reciprocal relationships, if the temperature is higher than the transcrystallization temperature, crystal form A is stable, and if the temperature is lower than the transcrystallization temperature, crystal form D is stable.From the following Table 9 to Table 10 the DSC data of the crystal forms A and D, it can be seen that TA > TD, ΔHf, A <ΔHf, D, i.e., the crystal form D is more stable at lower temperatures.

**Table 9 Thermodynamic stability relationship table**

| Crystal form | crystal form A | crystal form D |
|---|---|---|
| Melting point | TA | TD |
| Melting enthalpy | Δ Hf, A | Δ Hf, D |
| Thermodynami c stability | If: TA > TD; ΔHf, A >ΔHf, D crystal form A and crystal form D are univariate, and crystal form A is stable. | If: TA > TD; ΔHf, A <ΔHf, D crystal type A and crystal type D are reciprocal relationships, when the temperature is higher than the transcrystallization temperature T, crystal type A is stable; and when the temperature is lower than the transcrystallization temperature T, the crystal type D is stable. |

**Table 10 Summary of DSC data of sodium salt crystal form A and crystal form D**

| Cryst al form | Sampl eNo. | DSC endothermic peak (°C, peak temperature) | DSC exothermic peak (°C, peak temperature) | Melting enthalpy |
|---|---|---|---|---|
| A | TJA-II-033-1 | 144.89, 150.40 | 214.79 | 307.91 mJ |
| D | TJA-I-057-1 | ND | 210.79 | 1225.67 mJ |

### [Affinity test of recombinant Mcl-1 protein]

The binding affinity of the sodium salt D crystal form of TJ01-013 to the Mcl-1 protein is studied by an in vitro Surface Plasmon Resonance(SPR) test, using a Biacore analyzer and measuring with a CM5 chip being coupled to the protein. The experimental results are shown in Table 11 below.

**Table 11 Binding affinity of sodium salt D crystal form of TJ01-013 to Mcl-1 protein**

| | Protein | Affinity constantK D (M) | Binding constant ka (1/Ms) | Dissociation constant Kd (1/s) | Remarks |
|---|---|---|---|---|---|
| TJ0113 | Mcl-1 | 1.12E-06 | 1092.85714 3 | 0.001224 | specific binding |

Experiment conclusion: In SPR test, the sodium salt D crystal form of TJ01-013 can bind specifically to Mcl-1 protein.

### [Test for selective induction of autophagy indamaged mitochondrial inKeima HEK293 cell model]

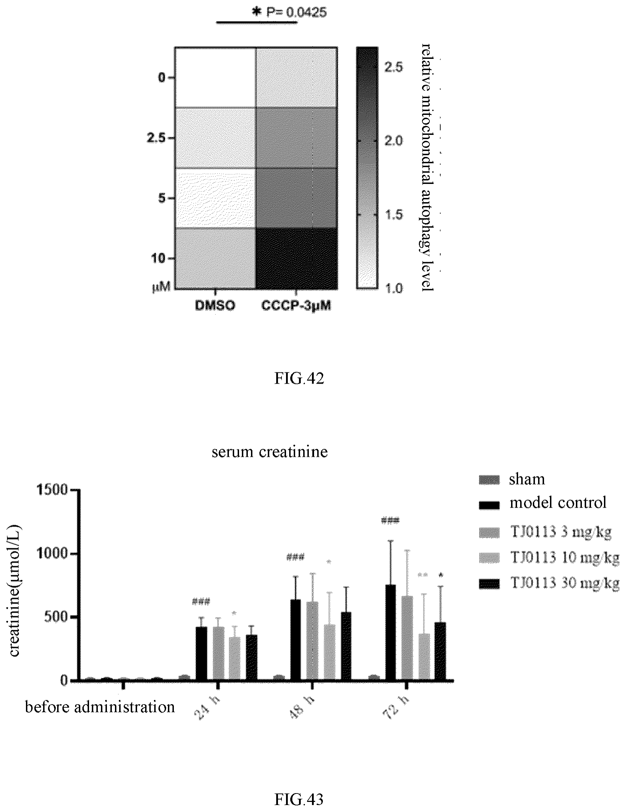

In HEK293T cells in vitro, fusing Keima fluorescent protein derived from the coral and the mitochondrial localization sequence of cytochrome C oxidase subunit IV asmtKeima protein, staining the nuclei with Hoechst33342 nuclear dye, and using the ThermoCellInsight^{™} CX7 LZR High Content Screening (HCS) platform at EX /EM=594nm/620nm conditions to assess mitophagy levels. The autophagy level of damaged mitochondria is tested by disrupting the mitochondrial membrane potential using 3 µMof Carbonyl cyanide 3-chlorophenylhydrazone (CCCP), causing mitochondrial damage. The results are shown in Fig.42, indicating that different concentrations of sodium salt D crystal from of TJ01-013 (0 µM, 2.5 µM, 5 µM, 10 µM) selectively induce autophagy in the damaged mitochondria (T test two-tails test P=0.0425), with good dose correlation and selectivity.

### [Rat pharmacokinetic test]

Rat Pharmacokinetic test of the sodium salt D crystal form of TJ01-013 .

The plasma kinetic characteristics of the prototype drugs in healthy SD rats are analyzed after three doses of single gavage administration, a single dose of consecutive multiple gavage administration, and a single dose of intravenous injection administration of the sodium salt D crystal form of TJ01-013. These include the rate of absorption and degree of exposure, individual differences, dose relationship, rate of elimination, attainment of steady state, and possible accumulation of D crystal form of TJ01-013.AfterSD rats are administrated 10, 30, and 90 mg/kg of D crystal form of TJ01-013 by gavage in a single dose, 30 mg/kg of D crystal form of TJ01-013 by gavage in a single dose once per day for 7 consecutive days, and 10 mg/kg of D crystal form of TJ01-013 by intravenous injection in a single dose, the data of the concentrations of the prototype drugs in plasma at different time points in each animal are calculated by applying WinNonlin software fitting, and the mean pharmacokinetic parameters of each group of 6 animals (half of males and half of females) are summarized as shown in Table 12 below.

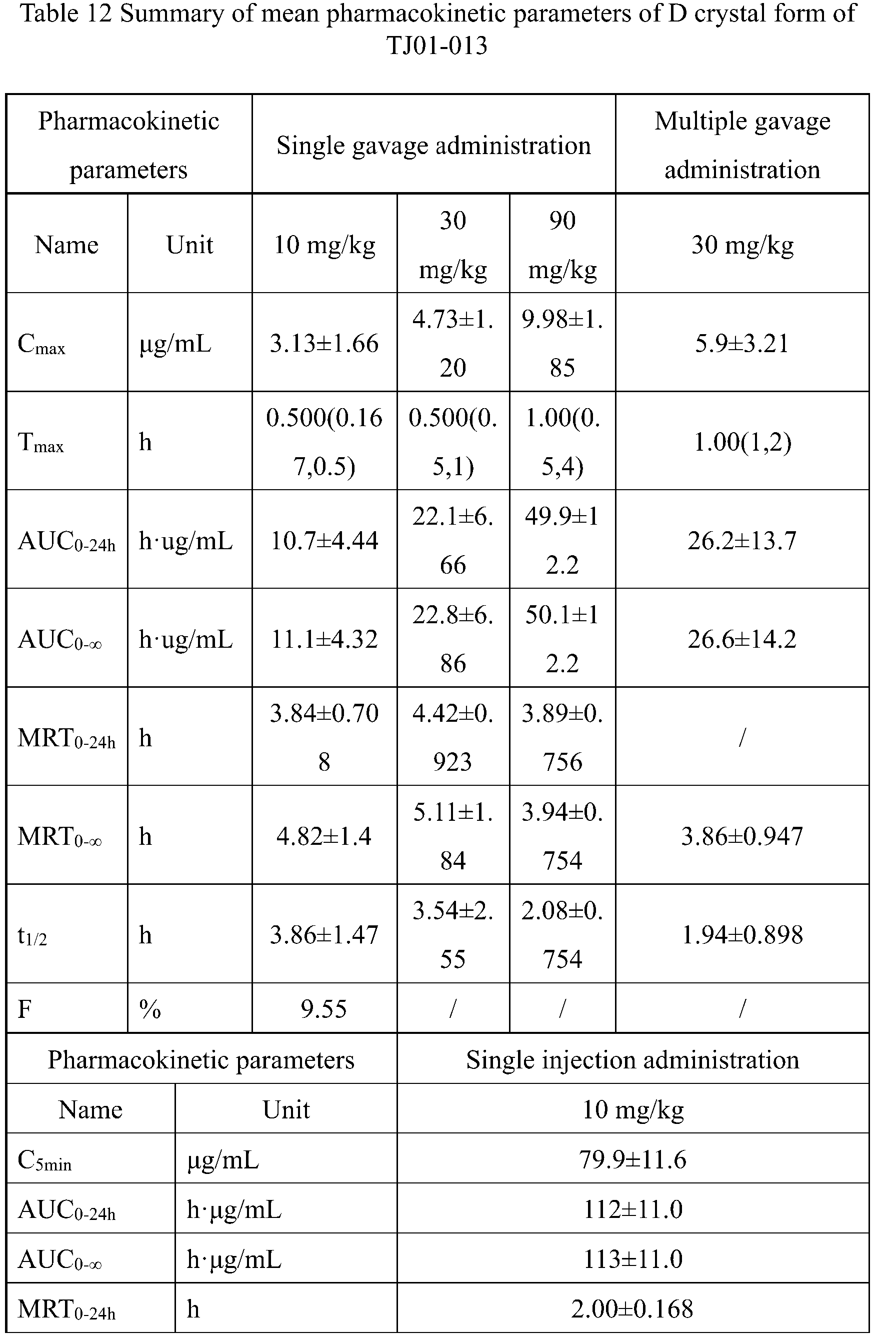

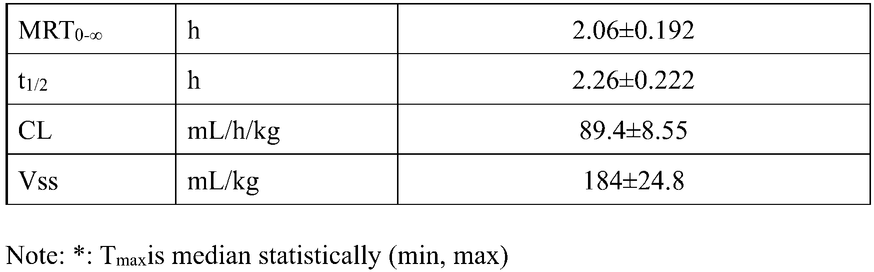

According to Table 12 above, after single gavage and intravenous administration of the D crystal form of TJ01-013 to SD rats, it shows the pharmacokinetic characteristics of rapid absorption, low apparent volume of distribution and moderate elimination rate.

After three doses of 10, 30, and 90 mg/kg of single gavage administration to SD rats, the exposureCₘₐₓ of the D crystal form of prototype TJ01-013 in plasma is 3.13±1.66, 4.73±1.20, and 9.98±1.85 µg/mL, respectively, the AUC₀₋₂₄ₕ is 10.7±4.44, 22.1±6.66, and 49.9±12.2 h-µg/mL, respectively, and increased with the dose increasing, and the increase ratio is smaller than that of the dose.

After 30 mg/kg of gavage administration once a day for 7 consecutive days to rats, the steady state is reached on the 4th day; compared with the gavage administration on the first day, the Cmax, AUC0-24h and Tmax on the 7th day are basically unchanged, and there is a tendency for the t_{1/2} to decrease, suggesting that there is no obvious accumulation when administered by gavage once a day for 7 consecutive days, but it shows tendency for elimination to become faster.

After single intravenous administration to SD rats, the Vssis 184 mL/kg suggesting that the D crystal form of TJ01-013 has a low degree tissue distribution. Compared with intravenous administration of the D crystal form of TJ01-013 at an equal dose, the absolute bioavailability of the prototype D crystal form of the TJ01-013 in SD rats administered 10 mg/kg by single gavage is 9.55%.

The plasma pharmacokinetic parameters of the prototype D crystal form of TJ01-013 are close to those of male and female animals after single intravenous injection, single gavage administration and multiple gavage administrations to SD rats, and there is no significant difference between male and female.

### [In vivo pharmacodynamic study: acute kidney injury model caused by unilateral renal ischemia and reperfusion in rats]

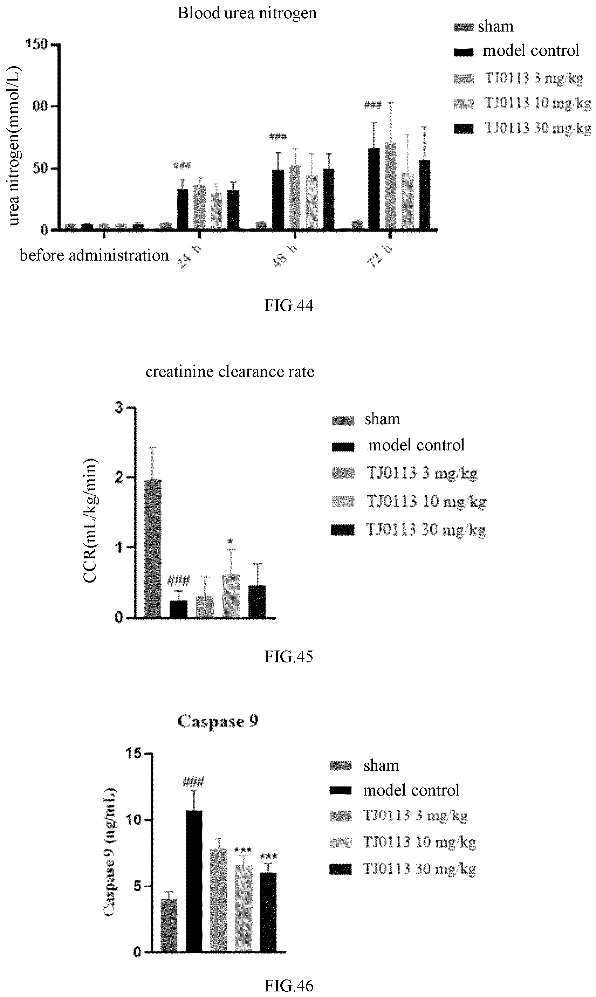

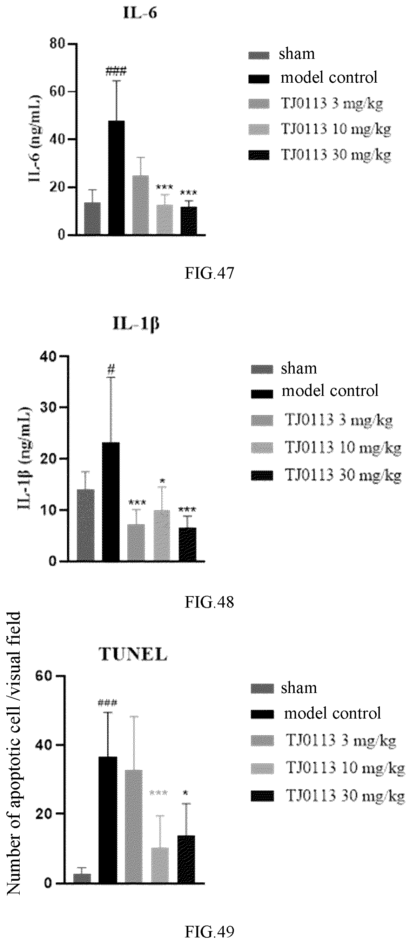

The pharmacodynamic effects of the D crystal form of TJ01-013 are studied in a renal ischemia-reperfusion (I/R) model of SD rats. Each group has twelve male rats of 280-300g. They are administered by gavage once a day for 3 consecutive days. Three dose groups are divided into 3 mg/kg, 10 mg/kg and 30 mg/kg. The sham group and modelcontrol group were administered by equal volumes of 0.5% CMC-Na, and left renal artery ligation is performed 1h afteradministration on the fourth day, and right nephrectomy is performed after 45 min of ischemia and before reperfusion, and then administered every day until 3 d after administration. The endpoint of the experiment is 3 d after administration. The results are shown in Fig. 43- 49.

Compared with the model-control group, the 3, 10, and 30 mg/kg group has no significant effect on body weight at 72 h after reperfusion. Compared with the model-control group, in the 10 mg/kg group, the creatinine values at 24 h, 48 h and 72 h after ischemia-reperfusion are significantly decreased, and the differences are statistically significant (P<0.05-0.01). The creatinine values at 24 h and 72 h after reperfusion in the 30 mg/kg group are also significantly decreased (P<0.05).There is a significant trend of decrease in urea nitrogen level at 72 h after ischemia-reperfusion in the 10 mg/kg and 30 mg/kg groups. Compared with the model control group, creatinine clearance rate in the 3 mg/kg, 10 mg/kg and 30 mg/kg groups show different degrees of improvement, among which the creatinine clearance rate in the 10 mg/kg group is statistically significant compared with that in the model control group (P<0.05). Compared with the model control group, the expression levels of apoptotic factor caspase 9 in renal tissues of the 10 mg/kg and 30 mg/kg groups are significantly decreased (P<0.001); and the expression levels of inflammatory factors IL-6 , IL-1β in renal tissues of the 3 mg/kg, 10 mg/kg, and 30 mg/kg groups are significantly decreased (P<0.05-0.001). Compared with the model control group, the apoptotic cell rate in renal tissue of the 10 mg/kg and 30 mg/kg groups is significantly decreased (P<0.001). Compared with the model control group, the degree of lesions of tubular dilatation, necrosis, glomerular atrophy, interstitial inflammatory cell infiltration, edema, fibrous tissue hyperplasia, dilatation of glomerular capillary bulb, dilatation of Bowman's capsule, and tubular mineralization in the 10 mg/kg group are significantly reduced, and 30 mg/kg shows a tendency to reduce the above lesions.

### [In vivo pharmacodynamic study: PAN-induced renal injury model in rats].

The effect of D crystal form of TJ01-013 on renal function is studied in the SD rat puromycin aminonucleoside (PAN) model. Each group has twelve male rats of 160-180g. On the first day of the experiment, except for the normal control group, rats in the other groupsare given a single intraperitoneal injection of 100mg/kg PAN (aminonucleoside puromycin) to induce the production of proteinuria. After modeling, the rats are randomly divided into the model control group, the three dose groups of 3 mg/kg, 10 mg/kg, and 30 mg/kg of the D crystal form of TJ01-013, and the normal control group. At the same time as intraperitoneal injection of PAN for modeling, the drug is administered by gavage once a day, with a volume of 10 mL/kg, for four consecutive weeks.

Compared with the model control group, the 3, 10, and 30 mg/kg groups show different degrees of reduction in urine protein concentration and the amount of the 24hurine protein on 7 and 12 d after administration, and show a certain trend of reduction in the amount of the 24hurine protein on 12 d after administration. Compared with the model control group, the 3, 10 and 30 mg/kg groups show different degrees of increase in blood albumin on 7d and 12 d after administration, among which the 3 mg/kg group is statistically significant(P<0.05). The 3, 10, and 30 mg/kg groups can reduce the urine protein concentration and the amount of the 24hurine protein on 7 and 12 d after administration in different degrees, and there is a certain trend of reduction in the amount of the 24hurine protein on 12 d after administration. Andthey cause different degrees of increase in blood albumin on 7d and 12 d after administration.

It can be seen that the gavage administration of 3, 10, 30 mg/kg of the D crystal form of TJ01-013 has a significant therapeutic effect on PAN-induced renal injury in rats. Pathologic microscopy results show that the low, medium and high dose groups of the tested drug reduce the degree of lesions of tubular dilatation, basophilic changes, hyaline tubular pattern, glomerular atrophy, Bowman's capsule dilatation, capsule wall thickening and interstitial inflammatory cell infiltration.

It can be understood by those of ordinary skill in the art that each of the above embodiments is a specific embodiment for realizing the present invention, and that in practical application, various changes can be made thereto in form and detail without departing from the spirit and scope of the present invention.

## Claims

1. A solid form of a pharmaceutically usable salt of a compound of formula (I);

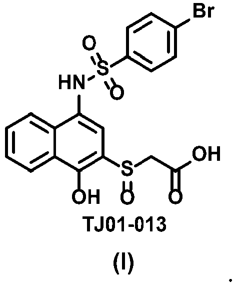

2. The solid form according to claim 1, wherein the solid form of a pharmaceutically usable salt is a sodium salt, a potassium salt, a calcium salt, an tromethamine salt, a lysine salt, a tert-butylamine salt, a diisopropylamine salt, an ethanolamine salt or a diethanolamine salt.

3. The solid form according to claim 1, wherein the solid form is a solid form of a sodium salt of a compound of formula (I), characteristic peaks of 2θ of 7.06 ° (±0.2° ) and 20.87 ° (±0.2° ) are showed in a X-ray powder diffraction pattern (Cu Kα rays) of the solid form of the sodium salt of the compound of formula (I).

4. The solid form according to claim 3, wherein at least one characteristic peak of 2θ selected from 7.06° (±0.2° ), 18.07° (±0.2° ), 25.02° (±0.2° ) and 20.87° (±0.2° ) is showed in the X-ray powder diffraction pattern of the solid form of the sodium salt of the compound of formula (I).

5. The solid form according to claim 3, wherein at least one characteristic peak of 2θ selected from 7.06° (±0.2° ), 18.07° (±0.2° ),25.02° (±0.2° ), 17.58° (±0.2° ), 20.87° (±0.2° ), 10.54° (±0.2° ), 23.91° (±0.2° ) is showed in the X-ray powder diffraction pattern of the solid form of the sodium salt of the compound of formula (I).

6. The solid form according to claim 3, wherein at least one characteristic peak of 2θ selected from 7.06° (±0.2° ), 18.07° (±0.2° ), 25.02 ° (±0.2° ), 17.58° (±0.2° ), 20.87° (±0.2° ), 10.54° (±0.2° ), 23.91 ° (±0.2 ° ), 27.65° (±0.2° ), 27.05° (±0.2° ), 21.68° (±0.2° ), and 25.91 ° (±0.2° ) is showed in the X-ray powder diffraction pattern of the solid form of the sodium salt of the compound of formula (I).

7. The solid form according to claim 3, wherein the solid form of the sodium salt of the compound of formula (I) shows the X-ray powder diffraction pattern substantially identical to that of Figure 10.

8. The solid form according to claim 3, wherein the solid form of the sodium salt of the compound of formula (I) further has at least one of the following features:
a differential scanning calorimetry curve of the solid form of the sodium salt of the compound of formula (I) has an endothermic peak at 183.79° C (± 3° C) and exhibits an exothermic peak at 210.79° C (± 3 ° C); and
a thermogravimetric analysis curve of the solid form of the sodium salt of the compound of formula (I) shows a thermogravimetric analysis curve pattern substantially identical to that of FIG. 12.

9. The solid form according to claim 1, wherein the solid form is a solid form of a potassium salt of the compound of formula (I), at least one characteristic peak of 2θ selected from 9.67° (±0.2° ) and 19.63° (±0.2° ) is showed in the X-ray powder diffraction profile he solid form of the potassium salt of the compound of formula (I) ;
and/or, the solid form is a solid form of a calcium salt of the compound of formula (I), at least one characteristic peak of 2θ selected from 11.53° (±0.2° ) and 21.54° (±0.2° ) is showed in the X-ray powder diffraction pattern of the solid form of the calcium salt of the compound of formula (I) ;
and/or, the solid form is a solid form of an tromethamine salt of the compound of formula (I), at least one characteristic peak of 2θ selected from 8.94° (±0.2° ) and 14.35° (±0.2° ) in the X-ray powder diffraction pattern of the solid form of the tromethamine salt of the compound of formula (I) ;
and/or, the solid form is a solid form of a lysine salt of the compound of formula (I), at least one characteristic peak selected of 2θ from 12.31 ° (±0.2° ) and 19.66° (±0.2° ) is showed in the X-ray powder diffraction pattern of the solid form of a lysine salt of the compound of formula (I) ;
and/or, the solid form is a solid form of a tert-butylamine salt of the compound of formula (I), at least one characteristic peak of 2θ selected from 7.15 ° (± 0.2 ° ) and 10.14 ° (± 0.2 ° ) is showed in the X-ray powder diffraction pattern of the solid form of the tert-butylamine salt of thecompound of formula (I);
and/or, the solid form is a solid form of a diisopropylamine salt of the compound of formula (I), at least one characteristic peak of 2θ selected from 8.87 ° ( ± 0.2 ° ) and 17.20 ° ( ± 0.2 ° ) is showed in the X-ray powder diffraction pattern of the solid form of the diisopropylamine salt of the compound of formula (I);
and/or, the solid form is a solid form of an ethanolamine salt of the compound of formula (I), at least one characteristic peak of 2θ selected from 7.62 ° (±0.2° ) and 19.70° (± 0.2 ° ) is showed in the X-ray powder diffraction pattern of the solid form of the ethanolamine salt of the compound of formula (I);
and/or, the solid form is a solid form of a diethanolamine salt of the compound of formula (I), at least one characteristic peak of 2θ selected from 6.33 ° (± 0.2 ° ) and 19.87 ° (± 0.2 ° ) is showed in the X-ray powder diffraction pattern of the solid form of a diethanolamine salt of a compound of formula (I).

10. A solid form of a free acid of a compound of formula (I); wherein at least one characteristic peak of 2θ selected from 8.92 ° (± 0.2 ° ) and 23.31° (± 0.2 ° ) is showed in a X-ray powder diffraction pattern of the solid form of the free acid of the compound of formula (I) ;

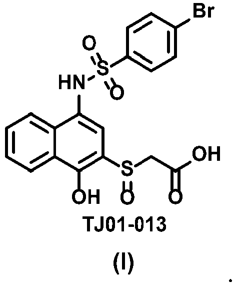

11. A method for preparing a solid form of a sodium salt of a compound of formula (I), wherein the method comprises the steps of: dissolving the compound of formula (I) in a reaction medium, and adding a base containing sodium and reacting to obtain.

12. A pharmaceutical composition, wherein the pharmaceutical composition includes a solid form of the compound of formula (I) according to any one of claims 1 to 10, and a pharmaceutically acceptable carrier or excipient.

13. Uses of a solid form of the compound of formula (I) according to any one of claims 1 to 10, or of a pharmaceutical composition according to claim 12, wherein the uses are for:
(i) preparing a drug for preventing and/or treating diseases associated with kidney injury; and/or
(ii) preventing and/or treating diseases associated with kidney injury; and/or
(iii) preventing and/or treating diseases associated with mitochondrial dysfunction; and/or
(iv) preparing a drug for preventing and/or treating diseases associated with mitochondrial dysfunction.
